# EUROPEAN PATENT SPECIFICATION

(11) **EP 2 694 658 B1**
(45) Date of publication and mention of the grant of the patent: **17.05.2017**
(21) Application number: 12712280.2
(22) Date of filing: 05.04.2012
(51) Int. Cl.: C12N 15/82, A01H 5/00

(54) **SEED-SPECIFIC PROMOTER IN COTTON**
SAMENSPEZIFISCHER PROMOTOR FÜR BAUMWOLLE
PROMOTEUR SPÉCIFIQUE DE GRAINE DANS LE COTON

(30) Priority: 07.04.2011 EP 11075060; 07.04.2011 US 201161472816 P
(43) Date of publication of application: 12.02.2014
(73) Proprietor: Bayer CropScience NV, 1831 Diegem (BE)
(72) Inventor: SCHEIRLINCK, Marie-Therese, 9620 Zottegem (BE); MEULEWAETER, Frank, 9820 Merelbeke (BE); JACOBS, John, 9820 Merelbeke (BE); VANDEWIELE, Martine, 9890 Dikkelvenne (BE); BOUDONCK, Kurt, Apex NC 27539 (US)
(86) International application number: PCT/EP2012/056324
(87) International publication number: WO 2012/136788

(56) References cited:
- WO-A2-2008/075364
- US-A- 5 495 070
- WANG SHUI ET AL: "Control of plant trichome development by a cotton fiber MYB gene", THE PLANT CELL, AMERICAN SOCIETY OF PLANT BIOLOGISTS, US, vol. 16, no. 9, 1 September 2004 (2004-09-01), pages 2323-2334, XP009088689, ISSN: 1040-4651, DOI: 10.1105/TPC.104.024844
- KIM H J ET AL: "A NOVEL EXPRESSION ASSAY SYSTEM FOR FIBER SPECIFIC PROMOTERS IN DEVELOPING COTTON FIBERS", PLANT MOLECULAR BIOLOGY REPORTER, NEW YORK, NY, US, vol. 20, no. 1, 1 March 2000 (2000-03-01), pages 7-18, XP009075630, ISSN: 0735-9640

### SEQUENCE LISTING

<110> Bayer CropScience N.V.
<120> Novel seed coat-specific promoter in cotton
<130> bcs11_2007
<160> 21
<170> PatentIn version 3.5
<210> 1
   <211> 1522
   <212> DNA
   <213> Gossypium hirsutum
<400> 1
   tatgtatatt atatattatt aataaaattg gaaataaact atatgtatga aattttttaa      60
   aaatattgag gccattaaag ttcatcaaaa cacatcattt ggcatcaaat taaacaaaac    120
   cttaaaatat atttagaaat atttaaaaaa atattgataa actttgatag attaatacca    180
   agctaaaatc aatagttccc ctaatgtagt agtactgaaa ttgtagaata ttatcttgat    240
   atggggttag actaatctta gtgggggtat tattacacag tgtacgtagt gcaggacaac    300
   gtgtatggca ccaaaaccct aactccatag aaatggaagg caaagataga gagtgtgaac    360
   gttgagtctg agtctaaaca ccgcccatta aatggagaca gtagaaaaga ttcgagaagg    420
   gcacgttagt gagttcaggg actttaaata aggattacaa aatatggaaa cctcattgct    480
   aaaaatggtg gaaatttgca cctgcaaagc tcttttcatt ttgctaattc tacaccctct    540
   tctctaaccc tttcggcaaa ttacaattcc aaggtctttt tccaactctg aaaacttgag    600
   tgagtctccc accccatctc tctaagctcc ctccctataa tatcacctat aagcttaggc    660
   ctaccctcct cggaggaaag ttattaaata tatactctca aaacactttg ctttctttgg    720
   gattcgtgat tttccgaaag ctgaattggc cggaactctt accaaaatac ctcaggtttg    780
   agggttttcg tttcttttcc tttcatcatc ttcttttaat gaagaaaaac aaaaacaatt    840
   gctctcagtt agatcttgtt aaatatgtat atacttactt tttttaactc ttatttcttt    900
   acctaaatgc atgctttctt tctttttttc tcatcttctt agatctgtgt tgtcagaacc    960
   ttctacttct ttgcatgatg aagatctgtt ctgttctatc tttcccttct tgcctttttt   1020
   cttcaccata atcatacaaa aatacatgat gggttctctt catatttcat gaatattcat   1080
   ccctacttgt gtttcagatc tttccaaaaa tcccttcaac ttgagctctt tttttttttg   1140
   tggagtaata atatgcaacg ataaaagtaa tagtacatcc atgaaataaa ttaaaaaccc   1200
   aaaagtcttt cttttcattt ttactaggcc tttagatttg taagtttctt ggaattgaag   1260
   ctagggtatg ggaataggaa tggctaactt ttcctccata cccaaaagtc ttttcttttc   1320
   tttttttttc agtctttatt atttctattt acgtctcaaa acaaatggat ctacttctta   1380
   tactctcaat ttccttcatc taaacaccat tttttttctc agattttcac tgattcatac   1440
   ttatcatcta tttcccaaaa ggtaaatatt attctaaaca ggcagatcgg gtagtgtttg   1500
   caggtacaag tgatcaaaga ga                                            1522
<210> 2
   <211> 20
   <212> DNA
   <213> Artificial Sequence
<220>
   <223> primer TSOL454_forward
<400> 2
   tcgaggccat acattctcag 20
<210> 3
   <211> 21
   <212> DNA
   <213> Artificial Sequence
<220>
   <223> primer TSOL455_reverse
<400> 3
   gtcttacaca ctctacacat c 21
<210> 4
   <211> 21
   <212> DNA
   <213> Artificial Sequence
<220>
   <223> primer TSOL456_forward
<400> 4
   agaggaactc ccactcccta c 21
<210> 5
   <211> 21
   <212> DNA
   <213> Artificial Sequence
<220>
   <223> primer TSOL457_reverse
<400> 5
   atgtagagta catatggttg a 21
<210> 6
   <211> 21
   <212> DNA
   <213> Artificial Sequence
<220>
   <223> primer TSOL458_forward
<400> 6
   catccatctg cttactccca t 21
<210> 7
   <211> 20
   <212> DNA
   <213> Artificial Sequence
<220>
   <223> primer TSOL459_reverse
<400> 7
   tacatcatac gaacttcaca 20
<210> 8
   <211> 22
   <212> DNA
   <213> Artificial Sequence
<220>
   <223> primer TSOL460_forward
<400> 8
   caaaccagct gatgcaagca gc 22
<210> 9
   <211> 22
   <212> DNA
   <213> Artificial Sequence
<220>
   <223> primer TSOL461_reverse
<400> 9
   caacaactag gctttcaact gt 22
<210> 10
   <211> 19
   <212> DNA
   <213> Artificial Sequence
<220>
   <223> primer TSOL472_forward
<400> 10
   ggtacaagtg atcaaagag 19
<210> 11
   <211> 20
   <212> DNA
   <213> Artificial Sequence
<220>
   <223> primer TSOL473_forward
<400> 11
   attggccgga actcttacca 20
<210> 12
   <211> 21
   <212> DNA
   <213> Artificial Sequence
<220>
   <223> primer TSOL465_reverse
<400> 12
   ggacctgatc ctagtaattc c 21
<210> 13
   <211> 20
   <212> DNA
   <213> Artificial Sequence
<220>
   <223> primer TSOL512_reverse
<400> 13
   agccattcct attcccatac 20
<210> 14
   <211> 22
   <212> DNA
   <213> Artificial Sequence
<220>
   <223> primer TSOL502
<400> 14
   ctgttctatc tttcccttct tg 22
<210> 15
   <211> 22
   <212> DNA
   <213> Artificial Sequence
<220>
   <223> primer TSOL503
<400> 15
   agaaagaaag catgcattta gg 22
<210> 16
   <211> 20
   <212> DNA
   <213> Artificial Sequence
<220>
   <223> primer TSOL500
<400> 16
   atacatgatg ggttctcttc 20
<210> 17
   <211> 20
   <212> DNA
   <213> Artificial Sequence
<220>
   <223> primer TSOL501
<400> 17
   aagcatgcat ttaggtaaag 20
<210> 18
   <211> 35
   <212> DNA
   <213> Artificial Sequence
<220>
   <223> primer TSOL558_forward
<400> 18
   gcgcggtacc gaattccata tgtatattat atatt 35
<210> 19
   <211> 27
   <212> DNA
   <213> Artificial Sequence
<220>
   <223> primer TSOL559_reverse
<400> 19
   tataactagt agtgtgctgg aattcgc 27
<210> 20
   <211> 28
   <212> DNA
   <213> Artificial Sequence
<220>
   <223> primer TSOL560_reverse
<400> 20
   gcatccatgg tctctttgat cacttgta 28
<210> 21
   <211> 1815
   <212> DNA
   <213> Artificial Sequence
<220>
   <223> nucleic acid sequence coding for beta-glucuronidase
<400> 21
   atggtaccgc gtactgtaga aaccccaacc cgtgaaatca aaaaactcga cggcctgtgg 60
   gcattcagtc tggatcgcga aaactgtgga attgatcagc gttggtggga aagcgcgtta 120
   caagaaagcc gggcaattgc tgtgccaggc agttttaacg atcagttcgc cgatgcagat 180
   attcgtaatt atgcgggcaa cgtctggtat cagcgcgaag tctttatacc gaaaggttgg 240
   gcaggccagc gtatcgtgct gcgtttcgat gcggtcactc attacggcaa agtgtgggtc 300
   aataatcagg aagtgatgga gcatcagggc ggctatacgc catttgaagc cgatgtcacg 360
   ccgtatgtta ttgccgggaa aagtgtacgt atcaccgttt gtgtgaacaa cgaactgaac 420
   tggcagacta tcccgccggg aatggtgatt accgacgaaa acggcaagaa aaagcagtct 480
   tacttccatg atttctttaa ctatgccgga atccatcgca gcgtaatgct ctacaccacg 540
   ccgaacacct gggtggacga tatcaccgtg gtgacgcatg tcgcgcaaga ctgtaaccac 600
   gcgtctgttg actggcaggt ggtggccaat ggtgatgtca gcgttgaact gcgtgatgcg 660
   gatcaacagg tggttgcaac tggacaaggc actagcggga ctttgcaagt ggtgaatccg 720
   cacctctggc aaccgggtga aggttatctc tatgaactgt gcgtcacagc caaaagccag 780
   acagagtgtg atatctaccc gcttcgcgtc ggcatccggt cagtggcagt gaagggcgaa 840
   cagttcctga ttaaccacaa accgttctac tttactggct ttggtcgtca tgaagatgcg 900
   gacttgcgtg gcaaaggatt cgataacgtg ctgatggtgc acgaccacgc attaatggac 960
   tggattgggg ccaactccta ccgtacctcg cattaccctt acgctgaaga gatgctcgac 1020
   tgggcagatg aacatggcat cgtggtgatt gatgaaactg ctgctgtcgg ctttaacctc 1080
   tctttaggca ttggtttcga agcgggcaac aagccgaaag aactgtacag cgaagaggca 1140
   gtcaacgggg aaactcagca agcgcactta caggcgatta aagagctgat agcgcgtgac 1200
   aaaaaccacc caagcgtggt gatgtggagt attgccaacg aaccggatac ccgtccgcaa 1260
   ggtgcacggg aatatttcgc gccactggcg gaagcaacgc gtaaactcga cccgacgcgt 1320
   ccgatcacct gcgtcaatgt aatgttctgc gacgctcaca ccgataccat cagcgatctc 1380
   tttgatgtgc tgtgcctgaa ccgttattac ggatggtatg tccaaagcgg cgatttggaa 1440
   acggcagaga aggtactgga aaaagaactt ctggcctggc aggagaaact gcatcagccg 1500
   attatcatca ccgaatacgg cgtggatacg ttagccgggc tgcactcaat gtacaccgac 1560
   atgtggagtg aagagtatca gtgtgcatgg ctggatatgt atcaccgcgt ctttgatcgc 1620
   gtcagcgccg tcgtcggtga acaggtatgg aatttcgccg attttgcgac ctcgcaaggc 1680
   atattgcgcg ttggcggtaa caagaaaggg atcttcactc gcgaccgcaa accgaagtcg 1740
   gcggcttttc tgctgcaaaa acgctggact ggcatgaact tcggtgaaaa accgcagcag 1800
   ggaggcaaac aatga 1815

## Description

The present application discloses a(n) (isolated) nucleic acid sequence comprising a nucleotide sequence selected from (a) SEQ ID NO: 1 or a fragment thereof, wherein said fragment comprises at least 600 consecutive nucleotides of SEQ ID NO: 1, the fragment further comprising a T/G motif having the nucleotide sequence from nucleotide position 298 to 303 of SEQ ID NO: 1 and a MYB binding motif having the nucleotide sequence from position 846 to 851 of SEQ ID NO: 1 and wherein the fragment when introduced into cotton has seed-specific promoter activity with high expression levels in fibers from day 5 post anthesis until day 30 post anthesis; (b) a nucleotide sequence with at least 95% sequence identity to the nucleotide sequence of (a), the fragment further comprising a T/G motif having the nucleotide sequence from nucleotide position 298 to 303 of SEQ ID NO: 1 and a MYB binding motif having the nucleotide sequence from position 846 to 851 of SEQ ID NO: 1 and having seed-specific promoter activity with high expression levels in fibers from day 5 post anthesis until day 30 post anthesis when introduced into cotton; and (c) a nucleotide sequence complementary to the nucleotide sequence of any one of (a) or (b). Further disclosed herein is a chimeric gene comprising the (isolated) nucleic acid described herein operably linked to a nucleic acid coding for an expression product of interest, and optionally a transcription termination and polyadenylation sequence. Also disclosed herein are a vector, a transgenic cotton plant cell, a transgenic cotton plant and a seed as characterized in the claims. Methods disclosed herein relate to effecting seed-specific expression of a product in cotton and of altering fiber properties in a cotton plant as characterized in the claims.

Trichomes are specialized epidermal appendages found on the surface of aerial organs of most land plants. There are several types of trichomes: unicellular or multicellular, branched or unbranched, and glandular or non-glandular. Trichomes contribute to many aspects of plant adaptation to biotic and abiotic stresses, such as to fence off insect herbivores, regulate surface temperature, decrease water loss through transpiration, increase tolerance to freezing, assist seed dispersal, and protect plant tissues from UV light (Eisner et al., 1998; Werker, 2000; Wagner et al., 2004). Glandular secreting trichomes (GSTs) often secrete plant secondary metabolites to constitute natural product-based resistance to herbivores and pathogens (Werker, 2000; Ranger and Hower, 2001; Wagner et al., 2004; Medeiros and Tingey, 2006).

Different plant species may have different types of trichomes, and one plant may form more than one type of trichomes. The annual weed *Arabidopsis thaliana* produces unicellular non-glandular trichomes, which may be branched or unbranched (Szymanski et al., 2000). Tobacco plants usually contain multicellular trichomes, including tall glandular secreting trichomes (GSTs) and simple glandless trichomes (Wagner et al., 2004). Cotton fibers are single-celled and extensively elongated seed trichomes (Kim and Triplett, 2001).

Cotton fiber is the single most important textile worldwide. About 80 million acres of cotton are harvested annually across the globe. Cotton is the fifth largest crop in the U.S. in terms of acreage production, with an average of 10.3 million acres planted in the years 2006 to 2008. About 90% of cotton grown worldwide is *Gossypium hirsutum,* whereas *Gossypium barbadense* accounts for about 8%. Consequently, the modification of cotton fiber characteristics to better suit the requirements of the industry and the consumer is a major effort in breeding by either classical methods or by genetically altering the genome of cotton plants. Goals to be achieved include increased lint fiber length, strength, dyability, decreased fuzz fiber production, fiber maturity ratio, immature fiber content, fiber uniformity and micronaire.

Cotton fiber development is a multistage process under the regulation of a vast number of genes, many of which are up-regulated or highly expressed in developing fiber cells (Li, C.H. et al., 2002; Ruan et al., 2003; Wang, S. et al., 2004; Li et al., 2005; Luo et al., 2007).

Various promoters driving expression of genes in the cotton seed have been described. Whereas seed-specific or trichome-specific promoters from cotton are known by now, also heterologous promoters are used to control seed-specific, seed-coat specific or trichome-specific expression in cotton.
E6 was the first cotton fiber gene identified, and the E6 promoter has been used for engineering cotton fiber quality (John and Keller, 1996). GhRDL1, a gene highly expressed in cotton fiber cells at the elongation stage, encodes a BURP domain containing protein (Li, C.H. et al., 2002), and the GaRDL1 promoter exhibited a trichome-specific activity in transgenic Arabidopsis plants (Wang, S. et al., 2004). GhTUB1 transcripts preferentially accumulate at high levels in fiber, accordingly, the pGhTUB1::GUS fusion gene was expressed at a high level in fiber but at much lower levels in other tissues (Li, X.B. et al., 2002). Promoters of three cotton lipid transfer protein genes, LTP3, LTP6, and FSItp4, were able to direct GUS gene expression in leaf and stem GSTs in transgenic tobacco plants (Hsu et al., 1999; Liu et al., 2000; Delaney et al., 2007), however, they did not exhibit a clear tissue-specificity. For example, in pFSItp4::GUS transgenic tobacco plants, strong GUS activity could be detected in all types of trichomes; in addition, GUS expression was also visible at the leaf margin, vascular tissue, ovules, and root tips (Delaney et al., 2007).

The cotton R2R3 MYB transcription factor GaMYB2 has been shown to be a functional homologue of Arabidopsis GLABRA1 (GL1), a key regulator of Arabidopsis trichome formation. GaMYB2 is expressed in cotton fiber cells at the early developmental stages (Wang, S. et al., 2004). Its promoter drives trichome-specific expression also in Arabidopsis and GST headspecific expression in tobacco (Shangguan et al., 2008).

US patent 7,626,081 discloses a cotton-seed specific promoter found in the alpha globulin gene. The promoter *Gh-sp* is derived from a seed protein gene and is active only in maturing cotton seeds (Song et al., 2000).

The FBP7 promoter from Petunia controls a MADS-box transcription factor and is known to be seed-specific. It has been shown that cotton plants transformed with a reporter construct driven by the FBP7 promoter specifically express said reporter in the seed coat (Pei et al., 2008).

Despite the fact that there are by now many promoters known to drive seed-specific, seed-coat specific or trichome-specific expression in cotton plants, it would be desirable to have further seed-specific, seed-coat specific or trichome-specific promoters available for seed-specific expression in cotton.

Accordingly, in one aspect, the present application discloses a(n) (isolated) nucleic acid sequence comprising a nucleotide sequence selected from (a) SEQ ID NO: 1 or a fragment thereof, wherein said fragment comprises at least 600 consecutive nucleotides of SEQ ID NO: 1, the fragment further comprising a T/G box motif having the nucleotide sequence from nucleotide position 298 to 303 of SEQ ID NO: 1 and a MYB binding motif having the nucleotide sequence from position 846 to 851 of SEQ ID NO: 1 and wherein the fragment when introduced into cotton has seed-specific promoter activity with high expression levels in fibers from day 5 post anthesis until day 30 post anthesis; (b) a nucleotide sequence with at least 95% sequence identity to the nucleotide sequence of (a) the fragment further comprising a T/G box motif having the nucleotide sequence from nucleotide position 298 to 303 of SEQ ID NO: 1 and a MYB binding motif having the nucleotide sequence from position 846 to 851 of SEQ ID NO: 1 and having seed-specific promoter activity with high expression levels in fibers from day 5 post anthesis until day 30 post anthesis when introduced into cotton and (c) a nucleotide sequence complementary to the nucleotide sequence of any one of (a) or (b).

The (isolated) nucleic acid sequence of this aspect is hereinafter also denoted the "promoter sequence".
Unless indicated otherwise, the embodiments described below for the promoter sequence disclosed herein are also applicable to respective embodiments of other aspects disclosed herein.

As used herein, the term "comprising" is to be interpreted as specifying the presence of the stated features, integers, steps or components as referred to, but does not preclude the presence or addition of one or more features, integers, steps or components, or groups thereof. Thus, e.g., a nucleic acid comprising a sequence of nucleotides, may comprise more nucleotides than the actually cited ones, i.e., be embedded in a larger nucleic acid. A chimeric gene as will be described further below which comprises a nucleic acid which is functionally or structurally defined may comprise additional nucleic acids etc. However, in context with the present disclosure, the term "comprising" also includes "consisting of".
In other words, the terminology relating to a nucleic acid "comprising" a certain nucleotide sequence or a protein comprising a certain amino acid sequence, as used throughout the text, refers to a nucleic acid or protein including or containing at least the described sequence, so that other nucleotide or amino acid sequences can be included at the 5' (or N-terminal) and/or 3' (or C-terminal) end, e.g. (the nucleotide sequence of) a selectable marker protein, (the nucleotide sequence of) a transit peptide, and/or a 5' leader sequence or a 3' trailer sequence.

Nucleic acids can be DNA or RNA, single- or double-stranded. Nucleic acids can be synthesized chemically or produced by biological expression in vitro or in vivo.
Nucleic acids can be chemically synthesized using appropriately protected ribonucleoside phosphoramidites and a conventional DNA/RNA synthesizer. Suppliers of RNA synthesis reagents are Proligo (Hamburg, Germany), Dharmacon Research (Lafayette, CO, USA), Pierce Chemical (part of Perbio Science, Rockford, IL , USA), Glen Research (Sterling, VA, USA), ChemGenes (Ashland, MA, USA), and Cruachem (Glasgow, UK).
In connection with the chimeric gene of the present disclosure, DNA includes cDNA and genomic DNA.

An "isolated nucleic acid" or "isolated nucleic acid sequence", as used in the present application, refers to a nucleic acid as defined above which is not naturally-occurring (such as an artificial or synthetic nucleic acid with a different nucleotide sequence than the naturally-occurring nucleic acid or a nucleic acid which is shorter than a naturally occurring one) or which is no longer in the natural environment wherein it was originally present, e.g., a nucleic acid coding sequence associated with a heterologous regulatory element (such as a bacterial coding sequence operably-linked to a plant-expressible promoter) in a chimeric gene or a nucleic acid transferred into another host cell, such as a transgenic plant cell.

The length of a fragment of SEQ ID NO: 1 as disclosed herein and its position within SEQ ID NO: 1 is to be chosen such that it is sufficiently long, e. g. comprising all elements necessary and sufficient, and positioned such that it is capable of inducing seed-specific, seed coat-specific or trichome-specific expression.
Methods of evaluating whether a nucleic acid sequence as described above, which in the present application represents a promoter sequence, is capable of inducing expression of coding sequence or a chimeric gene it is comprised in or, in particular, of a nucleic acid sequence operably linked thereto, in a seed-specific, seed coat-specific or trichome-specific manner are known to the skilled person.
For example reporter gene studies may be performed in order to evaluate the inducing function of a nucleic acid sequence. One example includes operably linking said first nucleic acid sequence to a reporter gene such as GUS, introducing the resulting nucleic acid construct in a plant or plant cell, such as in a cotton plant, and evaluating induction of the expression of said reporter gene in different tissues of said plant, as will also be described in more details further below.

Said fragment of the nucleic acid sequence described herein and having seed-specific, seed-coat-specific or trichome-/fiber-specific promoter activity in some examples may accordingly comprise at least 600, at least 650, at least 700, at least 800, at least 900, at least 1000, at least 1100, at least 1200, at least 1300 or at least 1400 consecutive nucleotides of SEQ ID NO: 1. In another example, said fragment comprises the nucleotide sequence from position 1 to position 748 of SEQ ID NO: 1, where position -1 is found. In another example, said fragment comprises the nucleotide sequence of SEQ ID NO: 1. In yet another example, said nucleic acid sequence consists of SEQ ID NO: 1.

However, it will be clear that variants of the present nucleotide sequence, including insertions, deletions and substitutions thereof may also be used to the same effect. Generally, such variants have at least 95% or even at least 98% sequence identity to SEQ ID NO: 1 and retain their seed-specific, seed coat-specific or trichome- or fiber-specific promoter activity.

As used herein, the term "promoter" denotes any nucleic acid sequence, such as DNA sequence, which is recognized and bound (directly or indirectly) by a DNA-dependent RNA-polymerase during initiation of transcription, resulting in the generation of an RNA molecule that is complementary to the transcribed DNA; this region may also be referred to as a "5' regulatory region". Promoters are usually located upstream of the 5' untranslated region (UTR) preceding the coding sequence to be transcribed and have regions that act as binding sites for RNA polymerase II and other proteins such as transcription factors to initiate transcription of an operably linked gene. Promoters may themselves contain sub-elements (i. e. promoter motifs) such as cis-elements or enhancer domains that regulate the transcription of operably linked genes. The promoter and a connected 5' UTR are also denoted as "promoter region".

A "seed-specific" promoter in the context of the present invention means that the transcription of a nucleic acid sequence controlled by a promoter is at least 5 times higher, at least 10 times higher, at least 20 times higher or at least 50 times higher in a seed cell than in cells of any other plant tissue.
In one example, seed-specific means seed-coat specific, i. e. no transcription takes place in gametophytically derived tissues of the seed. In another example, seed-specific or seed-coat specific means trichome-specific, i. e. no transcription takes place in parts of the seed or seed coat other than trichomes. Trichomes include fibers, e. g. of a cotton plant. Accordingly, the term "seed coat-specific" or "trichome-specific" means that the transcription of a nucleic acid sequence controlled by a promoter is effected such that transcription of said nucleic acid in the seed, the seed coat, the trichome or the fiber, respectively, is at least 5 times higher, at least 10 times higher, at least 20 times higher or at least 50 times higher than in cells of any other plant tissue, preferably plant tissue present during seed development such as during seed trichome development.
For the present invention, the promoter may also be seed-preferential. "Seed-preferential" expression (or "transcription" which is equivalent) in the context of this invention means the transcription of a nucleic acid sequence by a transcription regulating element such as a promoter in a way that transcription of said nucleic acid sequence in seeds contributes to more than 50%, preferably more than 60%, more preferably more than 70%, even more preferably more than 80% of the entire quantity of the RNA transcribed from said nucleic acid sequence in the entire plant during any of its developmental stages.

Confirmation of promoter activity for a promoter sequence or a functional promoter fragment may be determined by those skilled in the art, for example using a promoter-reporter construct comprising the promoter sequence operably linked to an easily scorable marker as herein further explained. The seed-specific, seed coat-specific or trichome-specific expression capacity of the identified or generated fragments or variants of the promoter described herein can be conveniently tested by operably linking such nucleic acid sequences to a nucleotide sequence encoding an easily scorable marker, e.g. a beta-glucuronidase gene, introducing such a chimeric gene into a plant and analyzing the expression pattern of the marker in seeds, the seed coat or trichomes as compared with the expression pattern of the marker in other parts of the plant. Candidates for a marker (or a reporter gene) other than the above-mentioned GUS are chloramphenicol acetyl transferase (CAT), beta-galactosidase (beta-GAL), and proteins with fluorescent or phosphorescent properties, such as green fluorescent protein (GFP) from Aequora Victoria or luciferase. To define a minimal promoter, a nucleic acid sequence representing the promoter is operably linked to the coding sequence of a marker (reporter) gene by recombinant DNA techniques well known to the art. The reporter gene is operably linked downstream of the promoter, so that transcripts initiating at the promoter proceed through the reporter gene. The expression cassette containing the reporter gene under the control of the promoter can be introduced into an appropriate cell type by transformation techniques well known in the art and described elsewhere in this application. To assay for the reporter protein, cell lysates are prepared and appropriate assays, which are well known in the art, for the reporter protein are performed. For example, if CAT were the reporter gene of choice, the lysates from cells transfected with constructs containing CAT under the control of a promoter under study are mixed with isotopically labeled chloramphenicol and acetyl-coenzyme A (acetyl-CoA). The CAT enzyme transfers the acetyl group from acetyl-CoA to the 2- or 3-position of chloramphenicol. The reaction is monitored by thin-layer chromatography, which separates acetylated chloramphenicol from unreacted material. The reaction products are then visualized by autoradiography. The level of enzyme activity corresponds to the amount of enzyme that was made, which in turn reveals the level of expression and the seed-specific, seed-coat specific or trichome-specific functionality of the promoter or fragment or variant thereof. This level of expression can also be compared to other promoters to determine the relative strength of the promoter under study. Once activity and functionality is confirmed, additional mutational and/or insertion and/or deletion analyses may be employed to determine e. g. a minimal region and/or sequences required to initiate transcription. Thus, sequences can be deleted at the 5' end of the promoter region and/or at the 3' end of the promoter region, or within the promoter sequence and/or nucleotide substitutions may be introduced. These constructs are then again introduced into cells and their activity and/or functionality are determined.
Instead of measuring the activity of a reporter enzyme, the transcriptional promoter activity (and functionality) can also be determined by measuring the level of RNA that is produced from the coding sequence operably linked to a promoter or fragment thereof. This level of RNA, such as mRNA, can be measured either at a single time point or at multiple time points and as such the fold increase can be average fold increase or an extrapolated value derived from experimentally measured values. As it is a comparison of levels, any method that measures mRNA levels can be used. In an example, the tissue or organs compared are a seed or seed tissue with a leaf or leaf tissue. In another example, multiple tissues or organs are compared. One example for multiple comparisons is a seed or seed tissue compared with 2, 3, 4, or more tissues or organs selected from the group consisting of floral tissue, floral apex, pollen, leaf, embryo, shoot, leaf primordia, shoot apex, root, root tip, vascular tissue and cotyledon. As used herein, examples of plant organs are seed, leaf, root, etc. and example of tissues are leaf primordia, shoot apex, vascular tissue, etc. The activity or strength of a promoter may be measured in terms of the amount of mRNA or protein accumulation it specifically produces, relative to the total amount of mRNA or protein. The promoter expresses an operably linked nucleic acid sequence for example at a level greater than about 0.1%, about 0.2%, greater than about 0.5, 0.6, 0.7, 0.8, or about 0.9%, greater than about 1 %, 2%, 3%, 4%, 5%, 6%, 7%, 8%, or about 9%, or greater than about 10% of the total mRNA of the cell it is contained in. Alternatively, the activity or strength of a promoter may be expressed relative to a well-characterized promoter (for which transcriptional activity was previously assessed) or the strength in a specific tissue may be expressed relative to that in another tissue.

Seed-specific, seed coat-specific or trichome-specific promoters are described which comprise a nucleotide sequence having at least 95% or at least 98% sequence identity to SEQ ID NO: 1 or a fragment thereof as defined above. Naturally occurring variants of the promoter disclosed herein can be identified with the use of well-known molecular biology techniques, as, for example, with polymerase chain reaction (PCR) and hybridization techniques as herein outlined before. Such nucleic acid sequences also include synthetically derived nucleic acid sequences, such as those generated, for example, by using site-directed mutagenesis of SEQ ID NO: 1 or a fragment thereof. Generally, nucleotide sequence variants will have at least 95%, 96%, 97%, to 98% and 99% sequence identity to the nucleic acid sequence of SEQ ID NO: 1. Derivatives of the nucleic acid sequences disclosed herein and having the required sequence identity may include, but are not limited to, deletions of sequences, single or multiple point mutations, alterations at a particular restriction enzyme recognition site, addition of functional elements, or other means of molecular modification which may enhance, or otherwise alter promoter expression. Techniques for obtaining such derivatives are well-known in the art (see, for example, J. F. Sambrook, D. W. Russell, and N. Irwin (2000) Molecular Cloning: A Laboratory Manual). For example, one of ordinary skill in the art may delimit the functional elements within the promoters disclosed herein and delete any non-essential elements. Functional elements may be modified or combined to increase the utility or expression of the sequences of the invention for any particular application. Those of skill in the art are familiar with the standard resource materials that describe specific conditions and procedures for the construction, manipulation, and isolation of macromolecules (e.g., DNA molecules, plasmids, etc.), as well as the generation of recombinant organisms and the screening and isolation of DNA molecules.
The promoter sequence of SEQ ID NO: 1 and its functional fragments and variants may for example be altered to contain e. g. "enhancer DNA" to assist in elevating gene expression. As is well-known in the art, certain DNA elements can be used to enhance the transcription of DNA. These enhancers are often found 5' to the start of transcription in a promoter that functions in eukaryotic cells, but can often be inserted upstream (5') or downstream (3') to the coding sequence. In some instances, these enhancer DNA elements are introns. Among the introns that are useful as enhancer DNA are the 5' introns from the rice actin 1 gene (see US5641876), the rice actin 2 gene, the maize alcohol dehydrogenase gene, the maize heat shock protein 70 gene (see US5593874), the maize shrunken 1 gene, the light sensitive 1 gene of Solanum tuberosum, and the heat shock protein 70 gene of Petunia hybrida (see US 5659122). Thus, as contemplated herein, a promoter or promoter region includes variations of promoters derived by inserting or deleting regulatory regions, subjecting the promoter to random or site-directed mutagenesis etc. The activity or strength of a promoter may be measured in terms of the amounts of RNA it produces, or the amount of protein accumulation in a cell or tissue, relative to a promoter whose transcriptional activity has been previously assessed, as described above.
As used herein, the term "percent sequence identity" refers to the percentage of identical nucleotides between two segments of a window of optimally aligned DNA. Optimal alignment of sequences for aligning a comparison window are well-known to those skilled in the art and may be conducted by tools such as the local homology algorithm of Smith and Waterman (Waterman, M. S., Chapman & Hall. London, 1995), the homology alignment algorithm of Needleman and Wunsch (1970), the search for similarity method of Pearson and Lipman (1988), and preferably by computerized implementations of these algorithms such as GAP, BESTFIT, FASTA, and TFASTA available as part of the GCG (Registered Trade Mark), Wisconsin Package (Registered Trade Mark from Accelrys Inc., San Diego, Calif.). An "identity fraction" for aligned segments of a test sequence and a reference sequence is the number of identical components that are shared by the two aligned sequences divided by the total number of components in the reference sequence segment, i.e., the entire reference sequence or a smaller defined part of the reference sequence. Percent sequence identity is represented as the identity fraction times 100. The comparison of one or more DNA sequences may be to a full-length DNA sequence or a portion thereof, or to a longer DNA sequence.

The term "hybridization" refers to the ability of a first strand of nucleic acid to join with a second strand via hydrogen bond base pairing when the two nucleic acid strands have sufficient sequence identity. Hybridization occurs when the two nucleic acid molecules anneal to one another under appropriate conditions. Nucleic acid hybridization is a technique well known to those of skill in the art of DNA manipulation. The hybridization property of a given pair of nucleic acids is an indication of their similarity or identity. Another indication that two nucleic acid sequences are largely identical is that the two molecules hybridize to each other under stringent conditions. "Stringent hybridization conditions" and "stringent hybridization wash conditions" in the context of nucleic acid hybridization experiments such as Southern and Northern hybridization are sequence dependent, and are different under different environmental parameters. An example of highly stringent wash conditions is 0.1 X SSC, 5X Denhardt's solution, 0.5% SDS at 65°C for e. g. about 15 minutes. An example of appropriate wash conditions for the present invention is a 2 X SSC, 0.1% SDS wash at 65°C for e. g. about 15 minutes. Often, a high stringency wash is preceded by a low stringency wash to remove background probe signal. Stringent conditions may also be achieved with the addition of destabilizing agents such as formamide. In general, a signal to noise ratio of 2 X (or higher) than that observed for an unrelated probe in the particular hybridization assay indicates detection of a specific hybridization. Very stringent conditions are selected to be equal to the Tm for a particular probe.

In the course of the present invention, a seed-specific promoter, together with a subsequent 5' UTR (together also denoted as "promoter region") has been identified in *Gossypium hirsutum.* Said promoter, also termed pMADS6 or GhpMADS6, in its natural context, controls the MADS box gene MADS6 in cotton.
*G*. *hirsutum* is an allotetraploid resulting from the fusion of two ancestral diploid species 1-1.2 million years ago and the genome can contain up to four alleles of each gene.

Considering that at least 100 MADS-box genes are present in the genome of *Arabidopsis* (Parenicová et al. 2003), with a similar number in poplar (Leseberg et al. 2006), petunia (Immink et al. 2003) and rice (Nam et al. 2004), the MADS-box gene family in cotton is likely to be large and complex, with a high level of homology and/or functional redundancy.
The promoter pMADS6 has a very low sequence identity of 46% to the petunia FBP7 promoter and is highly expressed in the ovule, fiber and in flower tissue. It has been shown that expression is particularly strong during early fiber development; expression of the gene product was detected to up to 24 DPA (Lightfoot et al., 2008).
It is expected that the current promoter is suitable for seed-specific, seed-coat specific or trichome- or fiber-specific expression or expression at least during early stages of developing fibers of transgenes in cotton. The current promoter can be used to express genes which modify cotton fiber properties or otherwise are involved in the formation of cotton fibers such as genes involved in auxin synthesis. The present promoter might also be easier controllable since it stems from the plant, into which it is intended to be reintroduced. Alternatively or in addition potential unknown or unwanted side-effects of heterologous promoters may be avoided by using the present promoter in cotton.

Unless indicated otherwise, the specific definitions or specific features of certain examples disclosed in the present application in connection with one aspect can be introduced into any other aspect disclosed herein.

A number of putative response elements were identified on the promoter sequence disclosed herein. The search was limited to trichome-specific elements and to a motif corresponding to a L1 box and MYB binding motives. The latter two have been described as motifs potentially conferring trichome-specific expression (Wang and Chen, 2004). The search revealed two motifs potentially conferring seed-specific, seed-coat specific or trichome-specific expression. The first one is a T/G box corresponding to the trichome motif RPSP01178 situated starting at position 298 in SEQ ID NO: 1 (corresponding to position -451). The exact sequence of the T/G box motif is AACGTG. Said binding motif has been identified in the promoter of a cotton fiber MYB gene (Shangguan et al., 2008) where deletion reduced the activity of the promoter in Arabidopsis and tobacco.

The other binding motif corresponds to a MYB binding motif and is found starting at position 846 in SEQ ID NO: 1 and has the sequence cagtta. Interestingly, said MYB binding motif is also present within the coding sequence of the MADS6 gene naturally regulated by the present promoter. It has been identified by Wang and Chen (2004) and is found in at least the RDL1 1 promoter where is confers trichome specificity in Arabidopsis, and in the GL1 (controlling the myb gene in Arabidopsis) and the GaMyb2 (controlling the MYB gene in cotton) promoters. It has been shown earlier that disruption of the MYB binding motif leads to a reduction in trichome production.

Variants of the promoter described herein include those which comprise both elements identified, but have otherwise been modified to delete nucleotide stretches within the sequence which are not needed for the promoter to be functional in a seed-specific, seed-coat specific or even trichome- or fiber-specific manner. For example, any nucleotide stretch located between both motives identified and/or between the transcriptional start and the first motif may be at least partially deleted to result in a shorter nucleotide sequence than the about 1,5 Kb sequence depicted in SEQ ID NO: 1.

The nucleotide sequence of the present promoter as well as fragments and variants thereof as defined above are expected to exert seed-specific, seed-coat specific or even trichome- or fiber-specific promoter activity.

In one example, the seed-specific promoter activity is in cotton. Other examples for which the promoter, fragments and variants thereof can be worked include other trichome- or fiber producing plants such as hemp, jute, flax and woody plants, including but not limited to *Pinus spp., Populus spp., Picea spp., Eucalyptus spp.* etc.

In one example of the nucleic acid sequence disclosed herein, the seed-specific promoter activity is trichome-specific or fiber-specific.

In another aspect, the present application discloses a chimeric gene comprising the (isolated) nucleic acid described herein operably linked to a nucleic acid coding for an expression product of interest, and optionally a transcription termination and polyadenylation sequence functional in plant cells.

A chimeric gene is an artificial gene constructed by operably linking fragments of unrelated genes or other nucleic acid sequences. In other words "chimeric gene" denotes a gene which is not normally found in a plant species or refers to any gene in which the promoter, adjoined parts of the promoter or one or more other regulatory regions of the gene are not associated in nature with a part or all of the transcribed nucleic acid operably linked therewith, i. e. are heterologous with respect to the transcribed nucleic acid. More particularly, a chimeric gene is an artificial, i. e. non-naturally occurring, gene produced by an operable linkage of the nucleic acid sequence of the invention, such as e. g. the nucleic acid of SEQ ID NO: 1, a 600 nt fragment thereof or a nucleic acid sequence having at least 95% sequence identity thereto, all capable of directing seed-specific, seed coat-specific or trichome-/fiber-specific expression of an expression product of interest as described above, with a second nucleic acid sequence encoding said expression product of interest which is not naturally operably linked to said nucleic acid sequence. Such nucleic acid sequence naturally operably linked to said nucleic acid sequence is the coding sequence of the cotton MADS6 gene.
The term "heterologous" refers to the relationship between two or more nucleic acid or protein sequences that are derived from different sources. For example, a promoter is heterologous with respect to an operably linked nucleic acid sequence, such as a coding sequence, if such a combination is not normally found in nature. In addition, a particular sequence may be "heterologous" with respect to a cell or organism into which it is inserted (i.e. does not naturally occur in that particular cell or organism). For example, the chimeric gene disclosed herein is a heterologous nucleic acid.

The term "operably linked" refers to the functional spatial arrangement of two or more nucleic acid regions or nucleic acid sequences. For example, a promoter region may be positioned relative to a nucleic acid sequence encoding an expression product of interest such that transcription of said nucleic acid sequence is directed by the promoter region. Thus, a promoter region is "operably linked" to the nucleic acid sequence.

The promoter, fragment or variant thereof as described above may be operably linked to a nucleic acid sequence encoding an expression product of interest that is heterologous with respect to the promoter. The nucleic acid sequence may generally be any nucleic acid sequence for which an altered level such as an increased level of transcription is desired. The nucleic acid sequence can for example encode a polypeptide that is capable of modifying fiber properties in cotton or involved in auxin biosynthesis.
Auxins are a class of plant hormones playing an essential role in coordination of many growth and behavioral processes in the plant life cycle. On the molecular level, auxins have an aromatic ring and a carboxylic acid group (Taiz and Zeiger, 1998). The most important member of the auxin family is indole-3-acetic acid (IAA). It generates the majority of auxin effects in intact plants, and is the most potent native auxin.

Further suitable heterologous nucleic acid sequences for modifying the properties of cotton fibers include, without limitation, those disclosed in WO02/45485 whereby fiber quality in fiber producing plants, such as cotton, is modified by modulating sucrose synthase activity and/or expression in such plants, the nucleic acids mediating an alteration of a fiber cell elongation phase by modulating deposition of callose as disclosed in WO2005/017157, in particular a gene encoding a β-1,3 glucan synthase protein, or in WO2006/136351.

An "expression product" denotes an intermediate or end product arising from the transcription and optionally translation of the nucleic acid, such as DNA or RNA, coding for such product. During the transcription process, a DNA sequence under control of regulatory regions, particularly the promoter sequence disclosed herein, is transcribed into an RNA molecule. An RNA molecule may either itself form an expression product and is then, for example, capable of interacting with another nucleic acid or protein. Alternatively, an RNA molecule may be an intermediate product when it is capable of being translated into a peptide or protein. A gene is said to encode an RNA molecule as expression product when the RNA as the end product of the expression of the gene is capable of interacting with another nucleic acid or protein. Examples of RNA expression products include inhibitory RNAs such as e. g. sense RNA, antisense RNA, hairpin RNA, ribozymes, miRNA or siRNA, mRNA, rRNA and tRNA. A gene is said to encode a protein or peptide as expression product when the end product of the expression of the gene is a protein or peptide.

Further exemplary expression products of interest include proteins involved in cell wall synthesis and fiber formation as disclosed in WO2005/017157, in particular a gene encoding a β-1,3 glucan synthase protein, or in WO2006/136351, PCT/EP2011/004929 or WO2011/089021, in particular an N-acetylglucosamine transferase which can be targeted to the membranes of the Golgi-apparatus, such as a N-acetylglucosamine transferase of the NODC type, or a chitin synthase.

Within the scope of the present disclosure, use may also be made, in combination with the chimeric gene described above, of other regulatory sequences, which are located between said nucleic acid sequence comprising a promoter and said nucleic acid sequence comprising the coding sequence of the expression product. This is especially the case if the nucleotide sequence used as promoter is the one from position 1 to position 748 of SEQ ID NO: 1 which corresponds to the promoter without 5'UTR. Non-limiting examples of such regulatory sequences include translation activators ("enhancers"), for instance the translation activator of the tobacco mosaic virus (TMV) described in Application WO 87/07644, or of the tobacco etch virus (TEV) described by Carrington & Freed 1990, J. Virol. 64: 1590-1597, or introns such as the Arabidopsis histon 3 intron (Chaubet *et al.,* 1992).
Other suitable regulatory sequences include 5' UTRs. As used herein, a 5'UTR, also referred to as leader sequence, is a particular region of a messenger RNA (mRNA) located between the transcription start site and the start codon of the coding region. It is involved in mRNA stability and translation efficiency. For example, the 5' untranslated leader of a petunia chlorophyll a/b binding protein gene (cab22L) downstream of the 35S transcription start site can be utilized to augment steady-state levels of reporter gene expression (Harpster et al., 1988, Mol Gen Genet. 212(1):182-90). WO95/006742 describes the use of 5' non-translated leader sequences derived from genes coding for heat shock proteins to increase transgene expression.

The chimeric gene may also comprise a transcription termination or polyadenylation sequence operable in a plant cell, particularly a cotton plant cell. As a transcription termination or polyadenylation sequence, use may be made of any corresponding sequence of bacterial origin, such as for example the nos terminator of *Agrobacterium tumefaciens,* of viral origin, such as for example the CaMV 35S terminator, or of plant origin, such as for example a histone terminator as described in published Patent Application EP 0 633 317 A1.

In one example of the chimeric gene described herein, said expression product of interest is a protein, a peptide or an RNA molecule, said RNA molecule capable of modulating the expression of a gene endogenous to said plant. In one example, said protein, peptide or RNA molecule is capable of modulating a fiber property. In another example, said protein, peptide or RNA molecule is involved in auxin biosynthesis.

The term "protein" as used herein describes a group of molecules consisting of more than 30 amino acids, whereas the term "peptide" describes molecules consisting of up to 30 amino acids. Proteins and peptides may further form dimers, trimers and higher oligomers, i.e. consisting of more than one (poly)peptide molecule. Protein or peptide molecules forming such dimers, trimers etc. may be identical or non-identical. The corresponding higher order structures are, consequently, termed homo- or heterodimers, homo- or heterotrimers etc. The terms "protein" and "peptide" also refer to naturally modified proteins or peptides wherein the modification is effected e.g. by glycosylation, acetylation, phosphorylation and the like. Such modifications are well known in the art.

Example proteins suitable as expression products include proteins involved in the modification of fiber properties, such as those mediating an increase in fiber length, an alteration in fiber strength or an alteration in the cell wall properties resulting, e. g. in an altered charge of said cell walls, an alteration in dyeability, decreased fuzz fiber production, an alteration in the fiber maturity ratio, a decrease in immature fiber content, or an increase in fiber uniformity and micronaire.

Said expression product of interest may also be an RNA molecule capable of modulating the expression of a gene endogenous to said cotton plant.

Examples of target genes suitable for RNA expression products in this connection include those involved in the modification of fiber properties, such as those mediating an increase in fiber length, an alteration in fiber strength or an alteration in the cell wall properties resulting, e. g. in an altered charge of said cell walls, an alteration in dyeability, decreased fuzz fiber production, an alteration in the fiber maturity ratio, a decrease in immature fiber content, or an increase in fiber uniformity and micronaire.

For the case of RNA molecules, it will be clear that whenever nucleotide sequences of RNA molecules are defined by reference to nucleotide sequence of corresponding DNA molecules, the thymine (T) in the nucleotide sequence should be replaced by uracil (U). Whether reference is made to RNA or DNA molecules will be clear from the context of the application.

The term "capable of modulating the expression of a gene" relates to the action of an RNA molecule, such as an inhibitory RNA molecule as described herein, to influence the expression level of target genes in different ways. This can be effected e. g. by inhibiting the expression of a target gene by directly interacting with components driving said expression such as the gene itself or the transcribed mRNA which results in a decrease of expression, or by inhibiting another gene involved in activating the expression of a target gene thereby abolishing said activation, or inhibiting another gene involved in inhibiting the expression of a target gene which results in an increase of expression. The inhibition of a gene involved in inhibiting the expression of a target gene using inhibitory RNA may, on the contrary, result in an activation of expression of said target gene.

Inhibitory RNA molecules decrease the levels of mRNAs of their target proteins available for translation into said target protein. In this way, expression of proteins involved in unwanted responses to stress conditions can be inhibited. This can be achieved through well established techniques including co-suppression (sense RNA suppression), antisense RNA, double-stranded RNA (dsRNA), siRNA or microRNA (miRNA).

An RNA molecule as expression product as disclosed herein comprises a part of a nucleotide sequence encoding a target protein or a homologous sequence to down-regulate the expression of said target protein. Another example for an RNA molecule as expression product for use in down-regulating expression are antisense RNA molecules comprising a nucleotide sequence complementary to at least a part of a nucleotide encoding a protein of interest or a homologous sequence. Here, down-regulation may be effected e. g. by introducing this antisense RNA or a chimeric DNA encoding such RNA molecule. In yet another example, expression of a protein of interest is down-regulated by introducing a double-stranded RNA molecule comprising a sense and an antisense RNA region corresponding to and respectively complementary to at least part of a gene sequence encoding said protein of interest, which sense and antisense RNA region are capable of forming a double stranded RNA region with each other. Such double-stranded RNA molecule may be encoded both by sense and antisense molecules as described above and by a single-stranded molecule being processed to form siRNA or miRNA.

In one example, expression of a target protein may be down-regulated by introducing a chimeric DNA construct which yields a sense RNA molecule capable of down-regulating expression by co-suppression. The transcribed DNA region will yield upon transcription a so-called sense RNA molecule capable of reducing the expression of a gene encoding a target protein in the target plant or plant cell in a transcriptional or post-transcriptional manner. The transcribed DNA region (and resulting RNA molecule) comprises at least 20 consecutive nucleotides having at least 95% sequence identity to the corresponding portion of the nucleotide sequence encoding the target protein present in the plant cell or plant.

Alternatively, an expression product for down-regulating expression of a target protein is an antisense RNA molecule. Down-regulating or reducing the expression of a protein of interest in the target cotton plant or plant cell is again effected in a transcriptional or post-transcriptional manner. The transcribed DNA region (and resulting RNA molecule) comprises at least 20 consecutive nucleotides having at least 95% sequence identity to the complement of the corresponding portion of the nucleic acid sequence encoding said target protein present in the plant cell or plant.

However, the minimum nucleotide sequence of the antisense or sense RNA region of about 20 nt of the nucleic acid sequence encoding a target protein may be comprised within a larger RNA molecule, varying in size from 20 nt to a length equal to the size of the target gene. The mentioned antisense or sense nucleotide regions may thus be from about 21 nt to about 5000 nt long, such as 21 nt, 40 nt, 50 nt, 100 nt, 200 nt, 300 nt, 500 nt, 1000 nt, 2000 nt or even about 5000 nt or larger in length. Moreover, it is not required for the purpose of the invention that the nucleotide sequence of the used inhibitory RNA molecule or the encoding region of the transgene, is completely identical or complementary to the endogenous gene encoding the target protein the expression of which is targeted to be reduced in the plant cell. The longer the sequence, the less stringent the requirement for the overall sequence identity is. Thus, the sense or antisense regions may have an overall sequence identity of about 40 % or 50 % or 60 % or 70 % or 80 % or 90 % or 100 % to the nucleotide sequence of an endogenous gene or the complement thereof. However, as mentioned, antisense or sense regions should comprise a nucleotide sequence of 20 consecutive nucleotides having about 95 to about 100 % sequence identity to the nucleotide sequence of the endogenous gene encoding the target gene. The stretch of about 95 to about 100% sequence identity may be about 50, 75 or 100 nt.

The efficiency of the above mentioned chimeric genes for antisense RNA or sense RNA-mediated gene expression level down-regulation may be further enhanced by inclusion of DNA elements which result in the expression of aberrant, non-polyadenylated inhibitory RNA molecules. One such DNA element suitable for that purpose is a DNA region encoding a self-splicing ribozyme. The efficiency may also be enhanced by providing the generated RNA molecules with nuclear localization or retention signals.

In addition, an expression product as described herein may be a nucleic acid sequence which yields a double-stranded RNA molecule capable of down-regulating expression of a gene encoding a target protein. Upon transcription of the DNA region the RNA is able to form dsRNA molecule through conventional base paring between a sense and antisense region, whereby the sense and antisense region are nucleotide sequences as hereinbefore described. Expression products being dsRNA according to the invention may further comprise an intron, such as a heterologous intron, located e.g. in the spacer sequence between the sense and antisense RNA regions in accordance with the disclosure of WO 99/53050. To achieve the construction of such a transgene, use can be made of the vectors described in WO 02/059294 A1.
In an example, said RNA molecule comprises a first and second RNA region wherein 1. said first RNA region comprises a nucleotide sequence of at least 19 consecutive nucleotides having at least about 94% sequence identity to the nucleotide sequence of said endogenous gene; 2. said second RNA region comprises a nucleotide sequence complementary to said 19 consecutive nucleotides of said first RNA region; 3. said first and second RNA region are capable of base-pairing to form a double stranded RNA molecule between at least said 19 consecutive nucleotides of said first and second region. In other examples, the same considerations apply as described above for sense and antisense RNA.

Another example expression product is a microRNA molecule (mirRNA, which may be processed from a pre-microRNA molecule) capable of guiding the cleavage of mRNA transcribed from the DNA encoding the target protein which is to be translated into said target protein. miRNA molecules may be conveniently introduced into plant cells through expression from a chimeric gene as described herein comprising a (second) nucleic acid sequence encoding as expression product of interest such miRNA, pre-miRNA or primary miRNA transcript.

miRNAs are small endogenous RNAs that regulate gene expression in plants, but also in other eukaryotes. As used herein, a "miRNA" is an RNA molecule of about 20 to 30 nucleotides (Siomi and Siomi, 2009) in length which can be loaded into a RISC complex and direct the cleavage of a target RNA molecule, wherein the target RNA molecule comprises a nucleotide sequence essentially complementary to the nucleotide sequence of the miRNA molecule. In example miRNAs, one or more of the following mismatches in the miRNA essentially complemntary to the target RNA may occur:
- A mismatch between the nucleotide at the 5' end of said miRNA and the corresponding nucleotide sequence in the target RNA molecule;
- A mismatch between any one of the nucleotides in position 1 to position 9 of said miRNA and the corresponding nucleotide sequence in the target RNA molecule;
- Three mismatches between any one of the nucleotides in position 12 to position 21 of said miRNA and the corresponding nucleotide sequence in the target RNA molecule provided that there are no more than two consecutive mismatches;
- No mismatch is allowed at positions 10 and 11 of the miRNA (all miRNA positions are indicated starting from the 5' end of the miRNA molecule).

A further example of an expression product capable of down-regulating expression of a target protein is encoded by a nucleic acid sequence which yields a pre-miRNA RNA molecule which is processed into a miRNA capable of guiding the cleavage of mRNA encoding said target protein. In plants, miRNAs are processed from the stem-loop regions of long endogenous pre-miRNAs by the cleavage activity of DICERLIKE1 (DCL1). Plant miRNAs are highly complementary to conserved target mRNAs, and guide the cleavage of their targets. miRNAs appear to be key components in regulating the gene expression of complex networks of pathways involved inter alia in development.

As used herein, a "pre-miRNA" molecule is an RNA molecule of about 100 to about 200 nucleotides, preferably about 100 to about 130 nucleotides which can adopt a secondary structure comprising a dsRNA stem and a single stranded RNA loop and further comprising the nucleotide sequence of the miRNA and its complement sequence of the miRNA* in the double-stranded RNA stem. Preferably, the miRNA and its complement are located about 10 to about 20 nucleotides from the free ends of the miRNA dsRNA stem. The length and sequence of the single stranded loop region are not critical and may vary considerably, e.g. between 30 and 50 nt in length. Preferably, the difference in free energy between unpaired and paired RNA structure is between -20 and -60 kcal/mole, for example around -40 kcal/mole. The complementarity between the miRNA and the miRNA* does not need to be perfect and about 1 to 3 bulges of unpaired nucleotides can be tolerated. The secondary structure adopted by an RNA molecule can be predicted by computer algorithms conventional in the art such as mFold, UNAFold and RNAFold. The particular strand of the dsRNA stem from the pre-miRNA which is released by DCL activity and loaded onto the RISC complex is determined by the degree of complementarity at the 5' end, whereby the strand which at its 5' end is the least involved in hydrogen bonding between the nucleotides of the different strands of the cleaved dsRNA stem is loaded onto the RISC complex and will determine the sequence specificity of the target RNA molecule degradation. However, if empirically the miRNA molecule from a particular synthetic pre-miRNA molecule is not functional because the "wrong" strand is loaded on the RISC complex, it will be immediately evident that this problem can be solved by exchanging the position of the miRNA molecule and its complement on the respective strands of the dsRNA stem of the pre-miRNA molecule. As is known in the art, binding between A and U involving two hydrogen bounds, or G and U involving two hydrogen bounds is less strong that between G and C involving three hydrogen bounds.

miRNA molecules may be comprised within their naturally occurring pre-miRNA molecules but they can also be introduced into existing pre-miRNA molecule scaffolds by exchanging the nucleotide sequence of the miRNA molecule normally processed from such existing pre-miRNA molecule for the nucleotide sequence of another miRNA of interest. The scaffold of the pre-miRNA can also be completely synthetic. Likewise, synthetic miRNA molecules may be comprised within, and processed from, existing pre-miRNA molecule scaffolds or synthetic pre-miRNA scaffolds.

Example expression products can also be ribozymes catalyzing either their own cleavage or the cleavage of other RNAs.

In one example of the chimeric gene disclosed herein modulating the expression is increasing the expression and said nucleic acid sequence encoding an expression product of interest encodes an RNA, which when transcribed 1. yields an RNA molecule capable of increasing the expression of a gene endogenous to said cotton plant. Such genes could be positively correlated with fiber length, fiber strength or a desired alteration in the cell wall properties resulting, e. g. in an altered charge of said cell walls, an alteration in dyeability, decreased fuzz fiber production, an alteration in the fiber maturity ratio, a decrease in immature fiber content, or an increase in fiber uniformity and micronaire, or 2. yields an RNA molecule capable of decreasing the expression of a gene endogenous to said cotton plant, wherein said gene may be negatively correlated with fiber length, fiber strength or a desired alteration in the cell wall properties resulting, e. g. in an altered charge of said cell walls, an alteration in dyeability, decreased fuzz fiber production, an alteration in the fiber maturity ratio, a decrease in immature fiber content, or an increase in fiber uniformity and micronaire.

Example RNA-based expression products include inhibitory RNAs such as miRNAs, siRNAs, antisense RNAs, sense RNAs, hairpin RNAs or ribozymes targeting glucanase, ADF encoding actin depolymerizing factor, CPC encoding caprice, or TRY encoding triptychon, among others.

In another example of the chimeric gene described herein, said expression product is a reporter gene or a fiber-specific gene as described elsewhere in this application.

In one example of the chimeric gene described herein, said RNA molecule comprises a first and second RNA region wherein 1. said first RNA region comprises a nucleotide sequence of at least 19 consecutive nucleotides having at least about 94% sequence identity to the nucleotide sequence of said endogenous gene; 2. said second RNA region comprises a nucleotide sequence complementary to said 19 consecutive nucleotides of said first RNA region; and 3. said first and second RNA region are capable of base-pairing to form a double stranded RNA molecule between at least said 19 consecutive nucleotides of said first and second region.

The present application also discloses a vector comprising the chimeric gene described herein.

A "vector" refers to any nucleic acid-based agent capable of carrying and transferring genetic information such as a plasmid, cosmid, virus, autonomously replicating sequence, phage, or linear single-stranded, circular single-stranded, linear double-stranded, or circular double-stranded DNA or RNA nucleotide sequence. The recombinant vector may be derived from any source and is capable of genomic integration or autonomous replication. Thus, the chimeric gene described above may be provided in a recombinant vector. A recombinant vector typically comprises, in a 5' to 3' orientation: a promoter to direct the transcription of a nucleic acid sequence and a nucleic acid sequence to be transcribed. These elements correspond to the chimeric gene disclosed herein to be introduced. The recombinant vector may further comprise a 3' transcriptional terminator, a 3' polyadenylation signal, other untranslated nucleic acid sequences, transit and targeting nucleic acid sequences, selectable markers, enhancers, and operators, as desired. The wording "5' UTR" refers to the untranslated region of DNA upstream, or 5' of the coding region of a gene and "3' UTR" refers to the untranslated region of DNA downstream, or 3' of the coding region of a gene. Means for preparing recombinant vectors are well known in the art. Methods for making recombinant vectors particularly suited to plant transformation are described in US4971908, US4940835, US4769061 and US4757011. The vector described herein may be an expression vector. Typical vectors useful for expression of nucleic acids in higher plants are well known in the art and include vectors derived from the tumor-inducing (Ti) plasmid of Agrobacterium tumefaciens.

In another aspect, the present application discloses a transgenic cotton plant cell comprising the chimeric gene disclosed herein or the vector disclosed herein.
The present invention is also directed to transgenic cotton plant cells and transgenic cotton plants which comprise a nucleic acid sequence as described above, i. e. the promoter sequence disclosed herein, operably linked to a heterologous nucleic acid sequence encoding an expression product of interest. Alternatively, said transgenic plant cells or plants comprise the chimeric gene disclosed herein. Preferred promoter sequences and expression products of interest and other regulatory elements, are described above.

A transgenic cotton plant may be produced by introducing the nucleic acid sequence(s) as described above into plants or plant cells. "Introducing" in connection with the present application relates to the placing of genetic information in a plant cell or plant by artificial means. This can be effected by any method known in the art for introducing RNA or DNA into plant cells, protoplasts, calli, roots, tubers, seeds, stems, leaves, seedlings, embryos, pollen and microspores, other plant tissues, or whole plants. More particularly, "introducing" means stably integrating into the plant's genome.
Plants containing transformed nucleic acid sequence are referred to as "transgenic plants". Transgenic and recombinant refer to a host organism such as a plant into which a heterologous nucleic acid molecule (e.g. the nucleic acid sequence, the chimeric gene or the vector as described herein) has been introduced. The nucleic acid can be stably integrated into the genome of the plant. Specific methods for introduction are described in connection with the methods disclosed herein.

"Cotton" or "cotton plant" as used herein can be any variety useful for growing cotton. The most commonly used cotton varieties are *Gossypium barbadense, G. hirsutum, G. arboreum* and *G*. *herbaceum.* Further varieties include G. africanum and G. raimondii. Also included are progeny from crosses of any of the above species with other species or crosses between such species.

A cotton plant cell may be any cell comprising essentially the genetic information necessary to define a cotton plant, which may, apart from the chimeric gene disclosed herein, be supplemented by one or more further transgenes. Cells may be derived from the various organs and/or tissues forming a cotton plant, including but not limited to fruits, seeds, embryos, reproductive tissue, meristematic regions, callus tissue, leaves, roots, shoots, flowers, vascular tissue, gametophytes, sporophytes, pollen, and microspores.

The present application also discloses a transgenic plant consisting of the transgenic cotton plant cell described hereinabove, or comprising the chimeric gene or the vector described herein stably integrated in the plant genome. This may be effected by transformation protocols described elsewhere in this application.

In another embodiment, the present invention relates to a seed generated from a transgenic cotton plant described herein, wherein said seed comprises the chimeric gene described herein.

Seed is formed by an embryonic plant enclosed together with stored nutrients by a seed coat. It is the product of the ripened ovule of gymnosperm and angiosperm plants, to the latter of which cotton belongs, which occurs after fertilization and to a certain extent growth within the mother plant.

Further disclosed herein are cotton fibers and cotton seed oil obtainable or obtained from the plants disclosed herein. Cotton fibers disclosed herein can be distinguished from other fibers by applying the detection method disclosed in WO2010/015423 and checking for the presence of the nucleic acid of (a) or chimeric gene of (b) in the fibers. Accordingly, the nucleic acid of (a) may also be used for tracking cell walls, in particular cotton fibers according to the invention.
Also disclosed herein are yarn and textiles made from the fibers disclosed herein as well as foodstuff and feed comprising or made of the cotton seed oil disclosed herein. A method to obtain cotton seed oil comprising harvesting cotton seeds from the cotton plant disclosed herein and extracting said oil from said seeds is also disclosed. Further, a method to produce cotton fibers comprising growing the cotton plant disclosed herein and harvesting cotton from said cotton plants is also disclosed.

A number of methods are available to introduce DNA into plant cells or plants, either by transformation or introgression. Agrobacterium-mediated transformation of cotton has been described e.g. in US patent 5,004,863, in US patent 6,483,013 and WO2000/71733.

Plants may also be transformed by particle bombardment: Particles of gold or tungsten are coated with DNA and then shot into young plant cells or plant embryos. This method also allows transformation of plant plastids. Cotton transformation by particle bombardment is reported e.g. in WO 92/15675.

Viral transformation (transduction) may be used for transient or stable expression of a gene, depending on the nature of the virus genome. The desired genetic material is packaged into a suitable plant virus and the modified virus is allowed to infect the plant. The progeny of the infected plants is virus free and also free of the inserted gene. Suitable methods for viral transformation are described or further detailed e. g. in WO 90/12107, WO 03/052108 or WO 2005/098004.

"Introgressing" means the integration of a gene in a plant's genome by natural means, i. e. by crossing a plant comprising the chimeric gene described herein with a plant not comprising said chimeric gene. The offspring can be selected for those comprising the chimeric gene.

Further transformation and introgression protocols can also be found in US patent 7,172,881.

In a further aspect, the present application discloses a method of growing cotton comprising (a1) providing the transgenic plant described herein or produced by the method described herein; or (a2) introducing a chimeric gene according described herein or a vector described herein in a plant; (b) growing the plant of (a1) or (a2); and (c) harvesting cotton produced by said plant.

"Growing" relates to creating the environment for plants to grow, multiply and/or age. Suitable growing conditions for specific plants are well-known in the art.

The present application also discloses a method of producing a seed comprising the chimeric gene disclosed herein comprising (a) growing a transgenic plant comprising the chimeric gene described herein or the vector described herein, a transgenic plant described herein or a transgenic plant obtained by the method described herein, wherein said transgenic plant produces said seed and said chimeric gene is comprised in said seed, and (b) isolating said seed from said transgenic plant.

In another aspect, the present application discloses to a method of effecting seed-specific expression of a product in cotton comprising introducing the chimeric gene disclosed herein or the vector disclosed herein into the genome of a cotton plant; or providing the transgenic plant disclosed herein. In one example, seed-specific expression may be seed coat-specific expression, trichome-specific expression or fiber-specific expression.

The present application further discloses a method of altering fiber properties in a cotton plant comprising introducing the chimeric gene disclosed herein or the vector disclosed herein into the genome of a cotton plant; or providing the transgenic plant disclosed herein.

In one example, the method further comprises growing said plant until seed are generated.

In another example based on the above further step, the method is for increasing cotton yield from a cotton plant and further comprises harvesting the cotton produced by said cotton plant. In other words disclosed herein is a method for increasing cotton yield from a cotton plant comprising introducing the chimeric gene disclosed herein or the vector disclosed herein into the genome of a cotton plant; or providing the transgenic plant disclosed herein; growing said plant until seed are generated; and harvesting the cotton produced by said cotton plant.

The term "increasing the yield" in connection with the present application relates to an increase in the output of cotton fibers which can be achieved e. g. by increasing the number of fibers produced on a cotton seed, the length of the fibers or the strength of the fibers. Genes and expression products thereof involved in conferring these properties have been described above.

In another aspect, the present application discloses the use of the chimeric gene disclosed herein, the vector disclosed herein or the transgenic plant or plant cell disclosed herein for seed-specific expression of a product in cotton, for altering fiber properties in cotton or for increasing cotton yield. The definitions and further examples described above for other aspects disclosed herein equally apply to the present aspect.

The transformed cotton plant cells and cotton plants disclosed herein or obtained by the methods described herein may contain, in addition to the chimeric gene described above, at least one other chimeric gene comprising a nucleic acid encoding an expression product of interest. Examples of such expression product include RNA molecules or proteins, such as for example an enzyme for resistance to a herbicide.
Further expression products of interest confer insect resistance to a cotton plant, i.e. resistance to attack by certain target insects, or tolerance to abiotic stresses.

The transformed plant cells and plants described herein such as those obtained by the methods described herein may be further used in breeding procedures well known in the art, such as crossing, selfing, and backcrossing. Breeding programs may involve crossing to generate an F1 (first filial) generation, followed by several generations of selfing (generating F2, F3, etc.). The breeding program may also involve backcrossing (BC) steps, whereby the offspring is backcrossed to one of the parental lines, termed the recurrent parent.
Accordingly, also disclosed herein is a method for producing plants comprising the chimeric gene disclosed herein comprising the step of crossing the cotton plant disclosed herein with another plant or with itself and selecting for offspring comprising said chimeric gene.

The transgenic plant cells and plants obtained by the methods disclosed herein may also be further used in subsequent transformation procedures, e. g. to introduce a further chimeric gene.

The figures show:
Figure 1: Agarose gel displaying results of PCR reaction to amplify DNA sequence of four MADS genes.
Figure 2: Scheme of inverse PCR procedure.

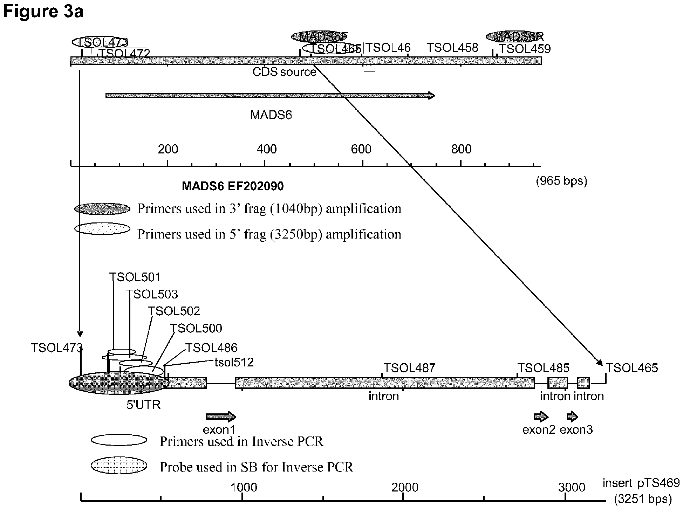

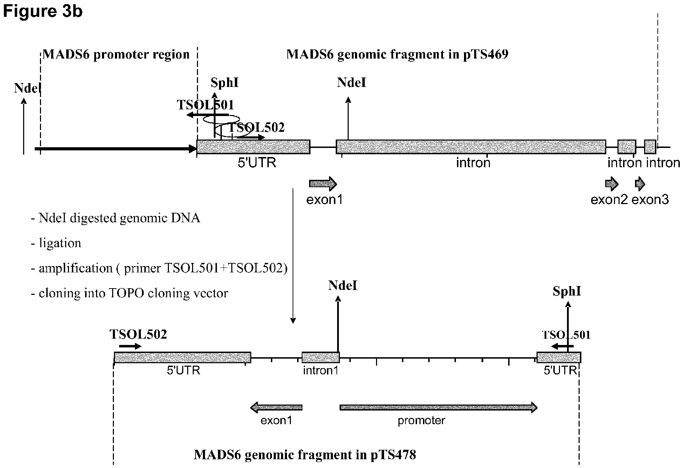

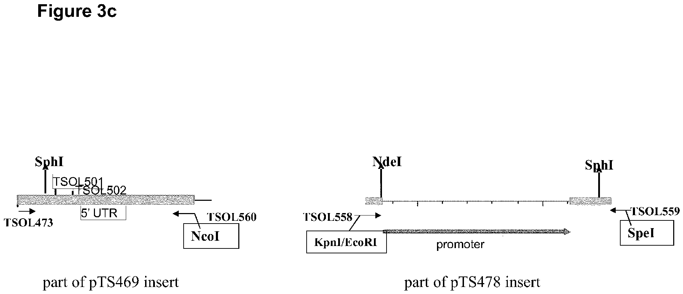

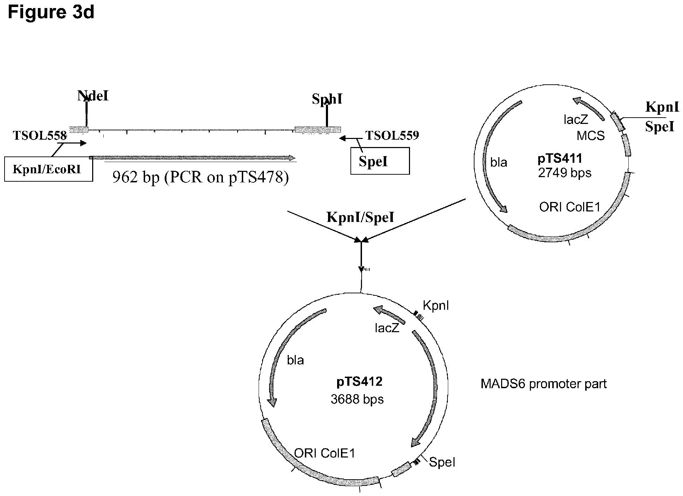

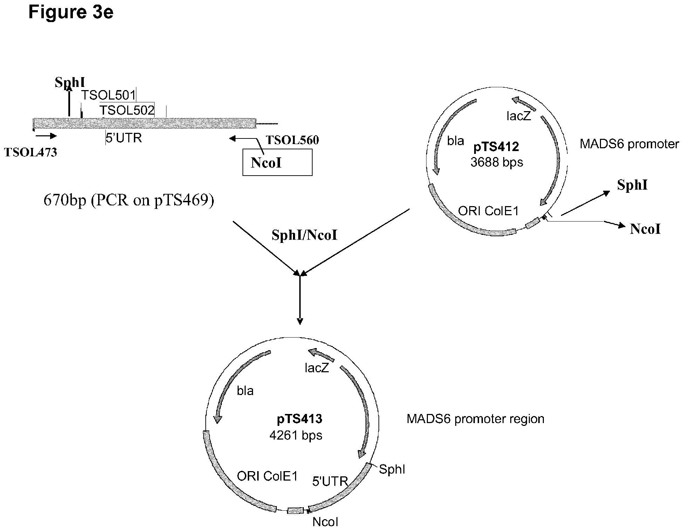

Figure 3: Cloning steps to retrieve the MADS6 promoter (PMADS6) as described in Example 1. 3a/3b Outline of retrieval of genomic sequence and inverse PCR approach; 3c. Fragments retrieved from inverse PCR; 3d to f: creation of vectors comprising complete MADS6 promoter. Abbreviations: bla: ampicillin resistance gene; ORI ColE1: Plasmid replication origin from pMB1; lacZ: Coding sequence encoding the beta-galactosidase alpha peptide from Escherichia coli; MCS: multi cloning site; 5' UTR: 5' untranslated region; Plac: Promoter of the Escherichia coli lac operon; Pmads6: MADS6 promoter; Phis: sequence including the promoter region of the histone H4 gene of Arabidopsis thaliana and the first intron of gene II of the histone H3.III variant of Arabidopsis thaliana; 2mepsps: coding sequence of the double-mutant 5-enol-pyruvylshikimate-3-phosphate synthase gene of Zea mays (corn) (Lebrun et al., 1997); TPotp C: coding sequence of the optimized transit peptide, containing sequence of the RuBisCO small subunit genes of Zea mays (corn) and Helianthus annuus (sunflower); aadA: Streptomycin and spectinomycin resistance; the coding sequence of the aminoglycoside adenyltransferase gene (aadA) of transposon Tn7 of Escherichia coli (Fling et al., 1985); bar: coding sequence of the phosphinothricin acetyltransferase gene (= bialaphos resistance gene) of Streptomyces hygroscopicus (Thompson et al., 1987); 3'nos: fragment of the 3' untranslated end of the nopaline synthase gene from the T-DNA of pTiT37 and containing plant polyadenylation signals; ori pVS1: Plasmid replication origin from pVS1 for stable maintenance in Agrobacterium; P35S3: Fragment of the promoter region from the Cauliflower Mosaic Virus 35S transcript; GUS: coding sequence of the beta-glucuronidase gene of Escherichia coli, including the second intron of the ST-LS1 gene of Solanum tuberosum (potato).
Figure 4: Making of cotton transformation vector pTTS108 comprising the putative MADS6 promoter, the GUS coding sequence and the bar selection marker.
Figure 5: Ovules transformed with the GUS reporter gene controlled by PMADS6 (Figure 5a) and a GUS reporter gene controlled by PFBP7 (Figure 5b) 6 dap. Circles indicate blue spots found on the ovules which corresponds to GUS expression.

The examples illustrate the invention.

### Materials

Unless indicated otherwise, chemicals and reagents in the examples were obtained from Sigma Chemical Company, restriction endonucleases were from Fermentas or Roche-Boehringer, and other modifying enzymes or kits regarding biochemicals and molecular biological assays were from Qiagen, Invitrogen and Q-BIOgene. Bacterial strains were from Invitrogen. The cloning steps carried out, such as, for example, restriction cleavages, agarose gel electrophoresis, purification of DNA fragments, linking DNA fragments, transformation of *E. coli* cells, growing bacteria, multiplying phages and sequence analysis of recombinant DNA, are carried out as described by Sambrook (1989). The sequencing of recombinant DNA molecules is carried out using ABI laser fluorescence DNA sequencer following the method of Sanger.

### Example 1: Tracking of MADS6 promoter in cotton

The petunia FBP7 promoter is known to express trichome-specifically. In the course of the present invention, a promoter with similar properties was identified in cotton.

A BLAST search with the sequence of the FBP7 protein controlled by the FBP7 promoter was effected in the TrEMBL database to identify potential homologs in cotton.

The search retrieved two hits in *Gossypium hirsutum*, both of which are MADS-box proteins termed GhMADS6 and GhMADS7.

In plants, MADS-box genes encode a large family of transcription factors of at least 100 members (reviewed by De Bodt et al. 2003; Kofuji et al. 2003; Parenicová et al. 2003; Nam et al. 2004).
GhMADS6 and GhMADS7, in addition to two more, GhMADS4 and GhMADS5, had been found to be homologs of FBP7 by Lightfoot et al. (2007). This group showed MADS5 and MADS6 to be highly expressed in the ovule, flower and fiber tissue. Furthermore, both proteins are expressed in early fiber development (0 to 6 DPA). No expression was detected in leafs and stem. MADS5 shows a low expression in the root.

In order to find a promoter specific for fibers, the gene encoding MADS6 was chosen for further investigation.

A blastn search was used to identify the cDNA encoding the MADS6 protein. The information retrieved revealed a cDNA fragment of 1040 base pairs. However, a search for this sequence in genomic databases did not produce any information. Accordingly, the nucleotide sequence 5' to the MADS6 coding sequence could not be determined.

In the present case, the MADS genes identified show a very high level of sequence identity. This makes tracking of the promoter of a specific MADS gene, in this case the MADS6 gene, a difficult task. For an approach based on inverse PCR, primers specific for the selected MADS gene need to be found which, considering the high sequence identity among the family members, is quite elaborate.

The genomic sequence was traced by PCR using the following primers.

### MADS4

TSOL454_forward: 5' - tcgaggccatacattctcag - 3' (SEQ ID NO: 2)
TSOL455_reverse: 5' - gtcttacacactctacacatc - 3' (SEQ ID NO: 3)

### MADS5

TSOL456_forward: 5' - agaggaactcccactccctac - 3'(SEQ ID NO: 4)
TSOL457_reverse: 5' - atgtagagtacatatggttga - 3' (SEQ ID NO: 5)

### MADS6

TSOL458_forward: 5' - catccatctgcttactcccat - 3' (SEQ ID NO: 6)
TSOL459_reverse: 5' - tacatcatacgaacttcaca - 3' (SEQ ID NO: 7)

### MADS7

TSOL460_forward: 5' - caaaccagctgatgcaagcagc - 3' (SEQ ID NO: 8)
TSOL461_reverse: 5' - caacaactaggctttcaactgt - 3' (SEQ ID NO: 9)

PCR reactions were performed on Cocker wild-type genomic DNA.

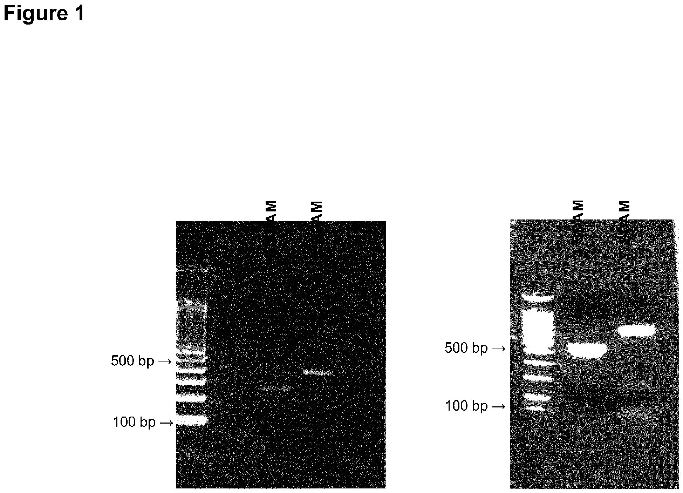

For all four genes, one single fragment larger than the expected fragment based on cDNA sequences was amplified (see Figure 1). This indicates that all four genes contain introns within the amplified coding region.

The PCR fragments obtained for MADS 4, MADS5 and MADS7 were stored at -20°C. The PCR fragment obtained for MADS6 was cloned into a cloning vector.

For comparison, a further PCR reaction was performed on genomic DNA for MADS6 using the primers reported in Lightfoot et al. (2007). Again, the fragment obtained (1040 bp) was larger than the corresponding cDNA fragment (394 bp). The fragment obtained was cloned and sequenced and aligned with a genomic fragment obtained with primers specific for the 3' end of MADS6, with the cDNA sequence of MADS6 and with the fragments obtained from cDNA using primers specific for the 3' end of MADS6.
Introns within the coding sequence could be confirmed.

The 1040 base pair fragment obtained corresponding to the 3' part of the MADS6 gene was then taken as a basis for amplification of the 5' genomic sequence comprising the promoter.

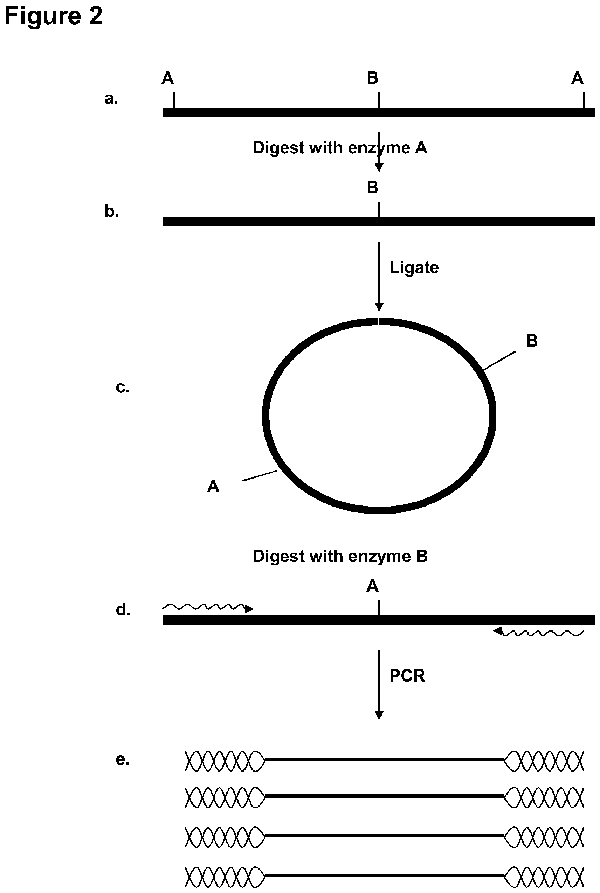

The inverse PCR approach was tried. An outline of the steps to be performed is depicted in figure 2.
A good candidate for enzyme A could be defined as Nhel. Trials with this enzyme resulted in fragments hybridizing to genomic fragments larger than 5 kb.

Then nested primers for inverse PCT were designed which target the 3' end of the MADS6 coding sequence which was found to be the least identical of all four MADS genes identified in *G. hirsutum.*

PCR reactions were set up with these primers and some candidates for enzyme A.

Only multiple non-specific bands could be obtained using the above primers specifically targeting MADS6 in the 3'-region of the gene, and the 5'-upstream sequence of the MADS6 gene could not be determined.

A further try was made with the less specific 5' sequence of the MADS6 gene. First, 5' - genomic sequences of MADS6 were amplified using forward primers primers TSOL472 (5' - GGTACAAGTGATCAAAGAG - 3'; SEQ ID NO: 10) resp. TSOL473 (5' - ATTGGCCGGAACTCTTACCA - 3'; SEQ ID NO: 11), binding to the 5'untranslated region (UTR) and reverse primer TSOL465 having the sequence 5' - GGACCTGATCCTAGTAATTCC - 3' (SEQ ID NO: 12) and binding to the MADS6 cDNA. Although the distance between TSOL472 and TSOL473 in the 5'UTR of the cDNA sequence is only 30bp, the amplified fragments (2500 bp resp. 3250 bp) differ by 750 bp in length; which indicates a 750 bp intron sequence in the 5'UTR of the MADS6 gene. Cloning and sequencing of both fragments confirmed this hypothesis.
The longer fragment of 3251 base pairs was taken as a basis for amplification of the 5' sequence comprising the promoter (see figure 3a).

In a further inverse PCR approach, a good candidate for enzyme B was defined by hybridization of digested genomic Coker DNA to a MADS6 5'UTR probe (540bp fragment obtained by PCR amplification with primers TSOL473 (SEQ ID NO: 11) and TSOL512 (5' - AGCCATTCCTATTCCCATAC - 3'; SEQ ID NO: 13).
For all restriction enzymes tested at least 2 hybridizing fragments were obtained, indicating cross-hybridization to at least one other MADS- family member.
From all enzymes, Bcll, Ndel and Hindlll were considered as most promising. All of these enzymes yielded 2 hybridizing bands of between 3.5 and 0.8 kb.

Next, Coker genomic DNA was digested in three reaction vials with Bcll, Ndel and Hindlll. 10µg digested genomic DNA, cleaned by precipitation and resuspend in 85µl TE buffer was self-ligated (in 100µl reaction volume) to obtain circular fragments (fig.3b) suitable for nested PCR analysis.

A nested PCR was performed using primers TSOL502 (5'-CTGTTCTATCTTTCCCTTCTTG - 3', SEQ ID NO: 14) and TSOL503 (5' - AGAAAGAAAGCATGCATTTAGG - 3'; SEQ IDNO: 15) for the first PCR reaction and primers TSOL500 (5'- ATACATGATGGGTTCTCTTC - 3'; SEQ ID NO: 16) and TSOL501 (5'- AAGCATGCATTTAGGTAAAG - 3'; SEQ ID NO: 17) for the second PCR reaction. The nested PCR resulted in the amplification of a band having a size corresponding to the hybridizing band for two of the three tested enzymes: amplification of a 850 bp fragment in Hindlll resp. a 1.7 kb fragment in Ndel digested and re-ligated genomic DNA. The amplified band of 1.7 kb was cloned into a cloning vector and sequenced. The recombinant vector was named pTS478 (Figure 3b).

The two fragments obtained and cloned which correspond to the 5'-UTR and the putative promoter region were joined as follows (Figure 3c).
As a first step both fragments were PCR amplified adding appropriate cloning sites for further cloning.
A/ Amplification of most upstream part: PCR amplification on pTS478 template DNA, using forward primer TSOL558 (5'-GCGCGGTACCGAATTCCATATGTATATTATATATT - 3' containing Kpnl and EcoRI restriction sites; SEQ ID NO: 18) and reverse primer TSOL559 (5'-TATAACTAGTAGTGTGCTGGAATTCGC - 3' containing a Spel restriction site; SEQ ID NO: 19). The fragment was cloned as intermediate vector (pTS412) and sequenced (Figure 3d).
B/ Amplification of the more 3' part of 5'UTR: PCR amplification on pTS469 template DNA, using forward primer TSOL473 (5'- ATTGGCCGGAACTCTTACCA- 3') and reverse primer TSOL560 (5'- GCATCCATGGTCTCTTTGATCACTTGTA- 3' containing a Ncol restriction site at the start of translation; SEQ ID NO: 20). After Sphl restriction digest, the fragment was ligated into pTS412, linearized by Sphl restriction digest. As a result, the two fragments could be joined in the vector pTS413; by this the complete (1.5 kb) 5'upstream sequence (promoter + 5'UTR including the 750bp intron) was reconstructed (Figure 3e). The sequence of the complete pTS413 insert (1.5kb) confirmed by sequencing.

For easy cloning and exchanging between different plant transformation vectors, the 1.5 kb MADS6 upstream fragment was cloned in a plasmid as Kpnl/Ncol fragment.

The resulting vector pTS414 contains a 1.5 kb fragment comprising the 5' upstream sequence of the MADS6 gene from *G. hirsutum* which comprises the putative promoter sequence

### Example 2: Construction of an expression cassette comprising the pMADS6 promoter and a sequence encoding an expression product of interest

The vector pTS414 comprising the 1.5 kb fragment obtained in example 1 and a vector comprising the coding sequence for an expression product of interest are digested with the appropriate restriction enzymes. The fragment of the sequence encoding the expression product of interest is ligated into the vector comprising said 1.5 kb fragment.

The resulting vector is then digested with appropriate restriction enzymes, the expression cassette comprising the sequenceencoding the expression product of interest joined to the putative MADS6 promoter iss purified and cloned into two vectors, one comprising the bar selection marker and one comprising the epsps selection.

### Example 3: Construction of an expression cassette comprising the pMADS6 promoter and the GUS reporter gene

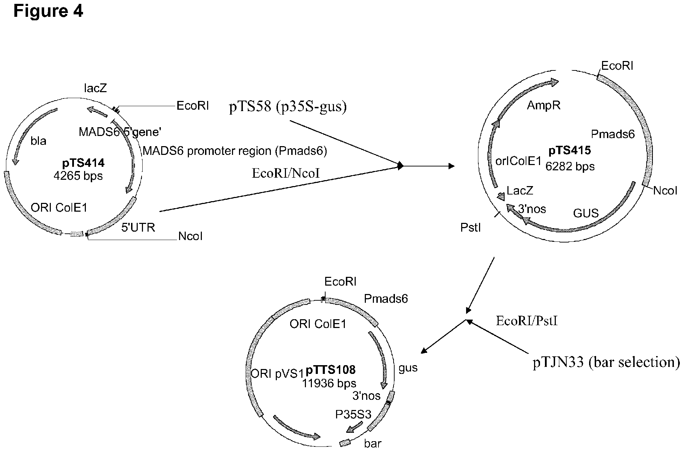

The vector pTS414 comprising the 1.5 kb fragment obtained in example 1 and a vector comprising the GUS coding sequence (SEQ ID NO: 21) were digested with restriction enzymes EcoRI and Ncol. The fragment of the GUS sequence was ligated into pTS414 to obtain pTS415 (see figure 4). A control construct was made by cloning the FBP7 promoter joined to the GUS reporter.

pTS415 was then digested with EcoRI and Pstl, the expression cassette comprising the GUS coding sequence joined to the putative MADS6 promoter was purified and cloned into a vector comprising the bar selection marker resulting in vector pTTS108 (Figure 4). This vector was used for cotton stable transformation.

### Example 4: Expression analysis of reporter constructs comprising pMADS6 and pFBP7, both joined to GUS reporter gene

Cotton ovules were transformed by particle bombardment using a Bio-RAD Model PDS-1000/He Biolistic Gene Gun. 60 mg gold particles of an average diameter of 0.3 to 3 µm were washed once with 70% ethanol and subsequently 3 times with distilled water. The particles were re-suspended in 1 ml distilled water. To 50 µl suspension, 5 µg DNA, 50 µl CaCl₂ and 20 µl spermidine were added, the resulting mixture was gently shaken for 10 min at RT and then left at RT for further 5 min. The resulting pellet was washed 3 times with 100% ethanol and then re-suspended in 55 µl 100% ethanol. 6 µl of the suspension were streaked on macrocarriers (Bio-RAD) and let dry. Particle bombardment was carried out using Rupture discs of 900,1100,1350 psi (Bio-RAD).

Expression of the GUS reporter gene controlled by PMADS6 (construct pTS417) or PFBP7 was analyzed 6 days after pollination (dap).

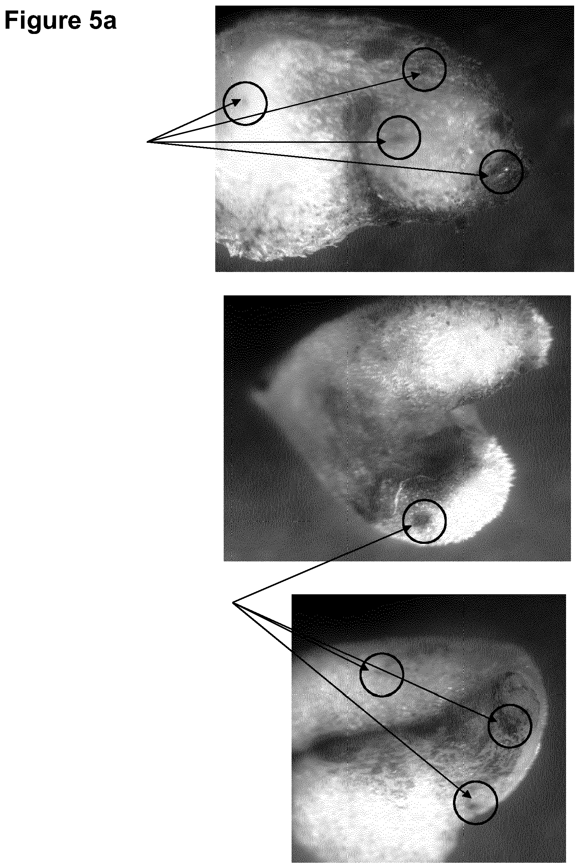

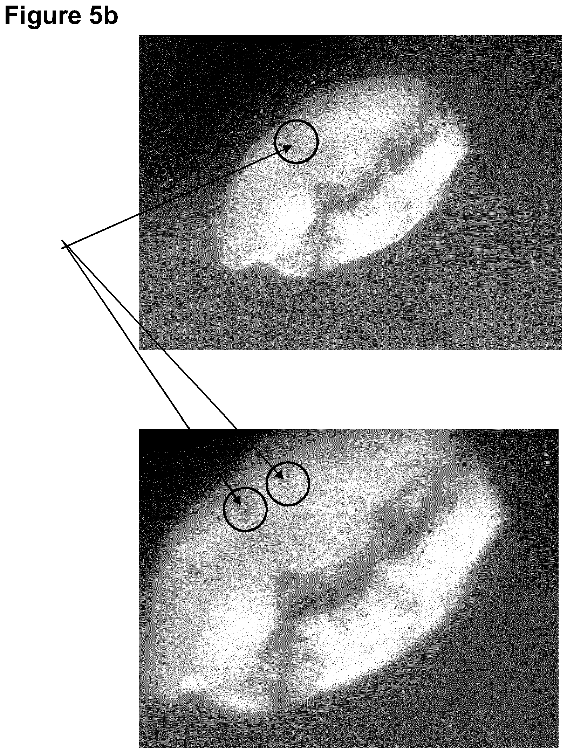

Figure 5 shows ovules transformed with the GUS reporter gene controlled by PMADS6 (Figure 5a) and a GUS reporter gene controlled by PFBP7 (Figure 5b) 6 dap. Circles indicate blue spots found on the ovules which corresponds to GUS expression. From these results, it is clear that PMADS6 drives expression in the ovule.

### Example 5: Analysis of pMADS6 for seed- and trichome-specific binding motives

A promoter analysis was carried out using publicly available databases such as PLACE (http://www.dna.affrc.go.jp/PLACE/), RegSite (http://linux1.softberry.com/berry.phtml?topic=regsitelist), PlantCare (Lescot et al., 2002; available at http://bioinformatics.psb.ugent.be/webtools/plantcare/html/) and AtcisDB (Davuluri et al., 2003).

The search was limited to trichome-specific elements as well as to a motives corresponding to a L1 box and MYB binding motives. The latter two have been described as motifs potentially conferring trichome-specific expression (Wang and Chen, 2004).

The search revealed two motifs potentially conferring seed-specific or seed-coat specific or trichome-specific expression. The first one is a T/G box corresponding to the trichome motif RPSP01178 situated starting from position -1224 (corresponding to position 298 of SEQ ID NO: 1. The exact sequence of the T/G box motif is AACGTG. Said binding motif has been identified in the promoter of a cotton fiber MYB gene (Shangguan et al., 2008) where deletion reduced the activity of the promoter in Arabidopsis and tobacco.
The other binding motif corresponds to a MYB binding motif and is found at position -676 (corresponding to position 846 of SEQ ID NO: 1. Interestingly, said MYB binding motif is also present in the coding sequence of the MADS6 gene naturally regulated by the present promoter. It has been identified by Wang and Chen (2004) and is found in at least the RDL1 promoter where is confers trichome specificity in Arabidopsis, and in the GL1 (controlling the myb gene in Arabidopsis) and the GaMyb2 (controlling the MYB gene in cotton) promoters. It has been shown earlier that disruption of the MYB binding motif leads to a reduction in trichome production.

### Example 6: expression analysis of pMADS6 in cotton fibers and other cotton plant tissues

Cotton was transformed by an Agrobacterium-mediated transformation method well-known in the art with the construct according to example 3 according to protocols well-known in the art and transformed plants were grown in the greenhouse. The plants were analyzed for GUS expression.

GUS expression in the developing fiber could be detected at 1 DPA, high expression at 5 DPA, 8 DPA, 10 DPA, 13 DPA and 30 DPA. Low GUS expression could be detected in the anthers. No expression was detected in sepal, stem, the floral axis and roots.

### References

Braasch and Corey (2001). Locked nucleic acid (LNA): fine-tuning the recognition of DNA and RNA. Chem Biol 8(1), p. 1-7.
Carrington & Freed 1990. Cap-independent enhancement of translation by a plant potyvirus 5' nontranslated region. J. Virol. 64, p. 1590-1597.
Chaubet et al. (1992). Genes encoding a histone H3.3-like variant in Arabidopsis contain intervening sequences. J Mol Biol. 225(2):569-74.
Davuluri et al. (2003). AGRIS: Arabidopsis gene regulatory information server, an information resource of Arabidopsis cis-regulatory elements and transcription factors. BMC Bioinformatics 4(1):25.
De Bodt, S. et al. (2003). Genomewide structural annotation and evolutionary analysis of the type I MADS-box genes in plants. J Mol Evol 56, p. 573-586.
Delaney et al. (2007). The fiber specificity of the cotton FSItp4 gene promoter is regulated by an AT-rich promoter region and the AT-hook transcription factor GhAT1. Plant and Cell Physiology 48, 1426-1437.
Eisner, T. et al. (1998). When defence backfires: detrimental effect of a plant's protective trichomes on an insect beneficial to the plant. Proc Natl Acad Sci USA 95, p. 4410-4414.
Hsu et al. (1999). Analysis of promoter activity of cotton lipid transfer protein gene LTP6 in transgenic tobacco plants. Plant Science 143, p. 63-70.
Immink, R. et al. (2003). Analysis of the petunia MADS-box transcription factor family. Mol Gen Genomics 268, p. 598-606.
John and Keller (1996). Metabolic pathway engineering in cotton: biosynthesis of polyhydroxybutyrate in fiber cell. Proceeding of the National Academy of Sciences, USA 93, 12678-12773.
Kim and Triplett, 82001). Cotton fiber growth in planta and in vitro. Models for plant cell elongation and cell wall biogenesis. Plant Physiology 127, p. 1361-1366.
Kofuji, R. et al. (2003). Evolution and divergence of teh MADS-box gene family base don genome-wide expression analysis. Mol Biol Evol 20, p. 1963.77.
Lescot et al. (2002). PlantCARE, a database of plant cis-acting regulatory elements and a portal to tools for in silico analysis of promoter sequences. Nucleic Acids Res. 2002 30(1):325-327.
Leseberg, C. et al. (2006). Genome-wide analysis of the MADS-box gene family in Populus trichocarpa. Gene 378, p. 84-94.
Li et al. (2005). The cotton ACTIN1 gene is functionally expressed in fibers and participates in fiber elongation. The Plant Cell 17, p. 859-875.
Li, C.H. et al. (2002). Isolation of genes preferentially expressed in cotton fibers by cDNA filter array and RT-PCR. Plant Science 163, p. 1113-1120.
Li, X.B. et al. (2002). Molecular characterization of the cotton GhTUB1 gene that is preferentially expressed in fiber. Plant Physiology 130, p. 666-674.
Lightfoot et al. (2008). Evidence for alternative splicing of MADS-box transcripts in developing cotton fiber cells. Mol Genet Genomics 279:75-85.
Liu et al. (2000). Cloning and promoter analysis of the cotton lipid transfer protein gene Ltp3. Biochimica et Biophysica Acta 1487, p. 106-111.
Luo et al. (2007). GhDET2, a steroid 5a-reductase, plays an important role in cotton fiber cell initiation and elongation. The Plant Journal 51, p. 419-430.
Medeiros and Tingey, (2006). Glandular trichomes of Solanum berthaultii and its hybrids with Solanum tuberosum affect nymphal emergence, development, and survival of Empoasca fabae (Homoptera: Cicadellidae). Journal of Economic Entomology 99, p. 1483-1489.
Nam, J. et al. (2004). Type I MADS-box genes have experienced faster birth- and death-evolution than type II MADS-box genes in angiosperms. PNAS 101, p. 1919-15.
Needleman and Wunsch (1970). A general method applicable to the search for similarities in the amino acid sequence of two proteins. J. Mol. Biol., 48, p.443-453.
Parenicová, L. et al. (2003). Molecular and phylogenetic analyses of the complete MADS-box transctiption factor family in Arabidopsis: new openings to the MADS world. Plant Cell 15, p. 1538-51.
Pearson and Lipman (1988). Improved tools for biological sequence comparison. Proc. Natl. Acad. Sci 85, p.2444-48.
Pei et al. (2008). Improvements of Fiber Yield and Fiber Fineness by Expressing the iaaM Gene in Cotton Seed Coat. Cotton Science 20, Supplement, p. 44.
Ranger and Hower, 2001. Role of the glandular trichomes in resistance of perennial alfalfa to the potato leafhopper (Homoptera: Cicadellidae). Journal of Economic Entomology 94, p. 950-957.
Ruan et al. (2003). Suppression of sucrose synthase gene expression represses cotton fiber cell initiation, elongation, and seed development. The Plant Cell 15, p. 952-964.
Sambrook, J.F., Russell, D.W. and Irwin, N. (2000). Molecular Cloning: A Laboratory Manual, 3rd edition Volumes 1 , 2, and 3. Cold Spring Harbor Laboratory Press.
Shangguan et al. (2008). Promoter of a cotton fiber MYB gene functional in trichomes of Arabidopsis and glandular trichomes of tobacco. Journal of Experimental Botany 59(13), p. 3533-3542.
Siomi, H. and Siomi, M. (2009). On the road to reading the RNA-interference code. Nature 457, 396-404.
Song et al. (2000). Expression of Two Tissue-Specific Promoters in Transgenic Cotton Plants. Journal of Cotton Science 4, p. 217-223.
Szymanski et al. (2000). Progress in the molecular genetic analysis of trichome initiation and morphogenesis in Arabidopsis. Trends in Plant Science 5, p. 214-219.
Wagner et al. (2004). New approaches for studying and exploiting an old protuberance, the plant trichome. Annals of Botany 93, p. 3-11.
Wang, S. et al. (2004). Control of plant trichome development by a cotton fiber MYB gene. The Plant Cell 16, p. 2323-2334.
Waterman, M. S. (1995). Introduction to Computational Biology: Maps, sequences and genomes. Chapman & Hall. London.
Werker, E. (2000). Trichome diversity and development. Advances in Botanical Research 31, p. 1-35.

## Claims

1. A nucleic acid sequence comprising a nucleotide sequence selected from
(a) SEQ ID NO: 1 or a fragment thereof, wherein said fragment comprises at least 600 consecutive nucleotides of SEQ ID NO: 1, said fragment further comprising a T/G box motif having the nucleotide sequence from nucleotide position 298 to 303 of SEQ ID NO: 1 and a MYB binding motif having the nucleotide sequence from nucleotide position 846 to 851 of SEQ ID NO: 1 and wherein said fragment when introduced into cotton has seed-specific promoter activity with high expression levels in fibers from day 5 post anthesis until day 30 post anthesis;
(b) a nucleotide sequence with at least 95% sequence identity to the nucleic acid sequence of (a), said fragment further comprising a T/G box motif having the nucleotide sequence from nucleotide position 298 to 303 of SEQ ID NO: 1 and a MYB binding motif having the nucleotide sequence from nucleotide position 846 to 851 of SEQ ID NO: 1 and having seed-specific promoter activity with high expression level in fibers from day 5 post anthesis until day 30 post anthesis when introduced in cotton;
(c) a nucleotide sequence complementary to the nucleotide sequence of any one of (a) or (b).

2. The nucleic acid of claim 1 , wherein said seed-specific promoter activity is trichome-specific.

3. A chimeric gene comprising the nucleic acid of claim 1 or claim 2 operably linked to a nucleic acid sequence encoding an expression product of interest, and optionally a transcription termination and polyadenylation sequence.

4. The chimeric gene of claim 3, wherein the said expression product of interest is a protein or an RNA molecule capable of modulating the expression of a gene endogenous to said plant.

5. The chimeric gene of claim 3 or 4, wherein said expression product is a reporter gene or a fiber-specific gene.

6. The chimeric gene of claim 4, wherein said RNA molecule comprises a first and second RNA region wherein
1. said first RNA region comprises a nucleotide sequence of at least 19 consecutive nucleotides having at least about 94% sequence identity to the nucleotide sequence of said endogenous gene;
2. said second RNA region comprises a nucleotide sequence complementary to said 19 consecutive nucleotides of said first RNA region; and
3. said first and second RNA region are capable of base-pairing to form a double stranded RNA molecule between at least said 19 consecutive nucleotides of said first and second region.

7. A vector comprising the chimeric gene of any one of claims 3 to 6.

8. A transgenic cotton plant cell comprising the chimeric gene of any one of claims 3 to 6 or the vector of claim 7.

9. A transgenic cotton plant comprising the chimeric gene of any one of claims 3 to 6 or the vector of claim 7 stably integrated in its genome or consisting of the transgenic cotton plant cell of claim 8 .

10. The transgenic plant of claim 9 which is G. hirsutum, G. barbadense, G. arboreum or G. herbaceum.

11. A seed generated from a transgenic plant according to claim 9 or 10, wherein the seed comprises the chimeric gene according to any one of claims 3 to 6.

12. Cotton fibers obtainable from the transgenic plant of claim 9 or 10.

13. A method of effecting seed-specific expression of a product in cotton comprising introducing the chimeric gene of any one of claims 3 to 6 or the vector of claim 7 into the genome of a cotton plant; or providing the transgenic plant of claim 9 or 10.

14. The method of claim 13, further comprising growing said plant until seeds are generated.

15. Use of the chimeric gene of any one of claims 3 to 6, the vector of claim 7 or the transgenic plant of claim 9 or 10 for seed-specific expression of a product in cotton, or for altering fiber properties in cotton.

## Patentansprüche

1. Nukleinsäuresequenz, umfassend eine Nukleotidsequenz, ausgewählt aus
(a) SEQ ID NO: 1 oder einem Fragment davon, wobei das Fragment mindestens 600 aufeinanderfolgende Nukleotide der SEQ ID NO: 1 umfasst, wobei das Fragment weiterhin ein T/G-Box-Motiv mit der Nukleotidsequenz von Nukleotidposition 298 bis 303 von SEQ ID NO: 1 und ein MYB-Bindungsmotiv mit der Nukleotidsequenz von Nukleotidposition 846 bis 851 von SEQ ID NO: 1 umfasst und wobei das Fragment, wenn es in Baumwolle eingeführt wird, eine samenspezifische Promoteraktivität mit hohen Expressionsniveaus in Fasern vom 5. Tag nach der Blüte bis zum 30. Tag nach der Blüte aufweist;
(b) einer Nukleotidsequenz mit mindestens 95% Sequenzidentität zu der Nukleinsäuresequenz von (a), wobei das Fragment weiterhin ein T/G-Box-Motiv mit der Nukleotidsequenz von Nukleotidposition 298 bis 303 von SEQ ID NO: 1 und ein MYB-Bindungsmotiv mit der Nukleotidsequenz von Nukleotidposition 846 bis 851 von SEQ ID NO: 1 umfasst und, wenn es in Baumwolle eingeführt wird, eine samenspezifische Promoteraktivität mit hohem Expressionsniveau in Fasern vom 5. Tag nach der Blüte bis zum 30. Tag nach der Blüte aufweist;
(c) einer Nukleotidsequenz, die zu der Nukleotidsequenz gemäß einem der Punkte (a) oder (b) komplementär ist.

2. Nukleinsäure nach Anspruch 1, wobei die samenspezifische Promoteraktivität trichomspezifisch ist.

3. Chimäres Gen, umfassend die Nukleinsäure nach Anspruch 1 oder Anspruch 2 in operativer Verknüpfung mit einer Nukleinsäuresequenz, die für ein Expressionsprodukt von Interesse codiert, sowie gegebenenfalls eine Transkriptionsterminations- und Polyadenylierungssequenz.

4. Chimäres Gen nach Anspruch 3, wobei es sich bei dem Expressionsprodukt von Interesse um ein Protein oder ein RNA-Molekül mit der Fähigkeit, die Expression eines in Bezug auf die Pflanze endogenen Gens zu modulieren, handelt.

5. Chimäres Gen nach Anspruch 3 oder 4, wobei es sich bei dem Expressionsprodukt um ein Reportergen oder ein faserspezifisches Gen handelt.

6. Chimäres Gen nach Anspruch 4, wobei das RNA-Molekül eine erste und zweite RNA-Region umfasst, wobei
1. die erste RNA-Region eine Nukleotidsequenz von mindestens 19 aufeinanderfolgenden Nukleotiden mit mindestens ungefähr 94% Sequenzidentität zu der Nukleotidsequenz des endogenen Gens umfasst;
2. die zweite RNA-Region eine Nukleotidsequenz, die zu den 19 aufeinanderfolgenden Nukleotiden der ersten RNA-Region komplementär ist, umfasst; und
3. die erste und zweite RNA-Region zur Basenpaarung befähigt sind, um zwischen mindestens den 19 aufeinanderfolgenden Nukleotiden der ersten und zweiten Region ein Doppelstrang-RNA-Molekül zu bilden.

7. Vektor, umfassend das chimäre Gen nach einem der Ansprüche 3 bis 6.

8. Transgene Baumwollpflanzenzelle, umfassend das chimäre Gen nach einem der Ansprüche 3 bis 6 oder den Vektor nach Anspruch 7.

9. Transgene Baumwollpflanze, umfassend das chimäre Gen nach einem der Ansprüche 3 bis 6 oder den Vektor nach Anspruch 7 stabil in ihr Genom integriert, oder bestehend aus der transgenen Baumwollpflanzenzelle nach Anspruch 8.

10. Transgene Pflanze nach Anspruch 9, bei der es sich um G. hirsutum, G. barbadense, G. arboreum oder G. herbaceum handelt.

11. Samen, der von einer transgenen Pflanze nach Anspruch 9 oder 10 produziert wird, wobei der Samen das chimäre Gen nach einem der Ansprüche 3 bis 6 umfasst.

12. Baumwollfasern, die von der transgenen Pflanze nach Anspruch 9 oder 10 erhältlich sind.

13. Verfahren zum Hervorrufen einer samenspezifischen Expression eines Produkts in Baumwolle, umfassend das Einführen des chimären Gens nach einem der Ansprüche 3 bis 6 oder des Vektors nach Anspruch 7 in das Genom einer Baumwollpflanze; oder Bereitstellen der transgenen Pflanze nach Anspruch 9 oder 10.

14. Verfahren nach Anspruch 13, weiterhin umfassend das Heranziehen der Pflanze, bis Samen produziert werden.

15. Verwendung des chimären Gens nach einem der Ansprüche 3 bis 6, des Vektors nach Anspruch 7 oder der transgenen Pflanze nach Anspruch 9 oder 10 für die samenspezifische Expression eines Produkts in Baumwolle bzw. zum Verändern der Fasereigenschaften in Baumwolle.

## Revendications

1. Séquence d'acide nucléique comprenant une séquence nucléotidique choisie parmi
(a) la SEQ ID n° : 1 ou un fragment de celle-ci, dans laquelle ledit fragment comprend au moins 600 nucléotides consécutifs de la SEQ ID n° : 1, ledit fragment comprenant en outre un motif de boîte T/G, ayant la séquence nucléotidique à partir de la position nucléotidique 298 jusqu'à la 303 de la SEQ ID n° : 1, et un motif de liaison MYB, ayant la séquence nucléotidique à partir de la position nucléotidique 846 jusqu'à la 851 de la SEQ ID n° : 1, et dans laquelle ledit fragment, quand il est introduit dans le coton, a une activité promotrice spécifique des graines, avec des niveaux d'expression élevés dans les fibres à partir du 5^{ème} jour après l'anthèse jusqu'au 30^{ème} jour après l'anthèse ;
(b) une séquence nucléotidique avec une identité de séquence d'au moins 95% avec la séquence d'acide nucléique du paragraphe (a), ledit fragment comprenant en outre un motif de boîte T/G, ayant la séquence nucléotidique à partir de la position nucléotidique 298 jusqu'à la 303 de la SEQ ID n° : 1, et un motif de liaison MYB, ayant la séquence nucléotidique à partir de la position nucléotidique 846 jusqu'à la 851 de la SEQ ID n° : 1, et ayant une activité promotrice spécifique des graines avec un niveau d'expression élevé dans les fibres à partir du 5^{ème} jour après l'anthèse jusqu'au 30^{ème} jour après l'anthèse quand il est introduit dans le coton ;
(c) une séquence nucléotidique complémentaire à la séquence nucléotidique selon l'un quelconque des paragraphes (a) ou (b).

2. Acide nucléique selon la revendication 1, dans lequel ladite activité promotrice spécifique des graines est spécifique des trichomes.

3. Gène chimère comprenant l'acide nucléique, selon la revendication 1 ou la revendication 2, étant lié, de manière opérationnelle, à une séquence d'acide nucléique qui code pour un produit d'expression d'intérêt et, en option, à une séquence de terminaison de la transcription et de polyadénylation.

4. Gène chimère selon la revendication 3, dans lequel ledit produit d'expression d'intérêt est une protéine ou une molécule d'ARN étant capable de moduler l'expression d'un gène endogène à ladite plante.

5. Gène chimère selon la revendication 3 ou 4, dans lequel ledit produit d'expression est un gène rapporteur ou un gène spécifique des fibres.

6. Gène chimère selon la revendication 4, dans lequel ladite molécule d'ARN comprend une première et une deuxième région d'ARN dans lequel
1. ladite première région d'ARN comprend une séquence nucléotidique, d'au moins 19 nucléotides consécutifs, ayant une identité de séquence d'au moins 94% environ avec la séquence nucléotidique dudit gène endogène ;
2. ladite deuxième région d'ARN comprend une séquence nucléotidique complémentaire auxdits 19 nucléotides consécutifs de ladite première région d'ARN ; et
3. ladite première et deuxième région d'ARN sont capables de s'apparier par bases pour former une molécule d'ARN en double brin entre au moins lesdits 19 nucléotides consécutifs de ladite première et deuxième région.

7. Vecteur comprenant le gène chimère selon l'une quelconque des revendications 3 à 6.

8. Cellule d'un plant de coton transgénique comprenant le gène chimère selon l'une quelconque des revendications 3 à 6 ou le vecteur selon la revendication 7.

9. Plant de coton transgénique comprenant le gène chimère selon l'une quelconque des revendications 3 à 6 ou le vecteur selon la revendication 7, stablement intégré dans son génome ou étant constitué par la cellule d'un plant de coton transgénique selon la revendication 8.

10. Plante transgénique selon la revendication 9, qui est G. hirsutum, G. barbadense, G. arboreum ou G. herbaceum.

11. Graine générée à partir d'une plante transgénique selon la revendication 9 ou 10, dans laquelle la graine comprend le gène chimère selon l'une quelconque des revendications 3 à 6.

12. Fibres de coton que l'on peut obtenir à partir de la plante transgénique selon la revendication 9 ou 10.

13. Procédé de réalisation de l'expression spécifique des graines d'un produit chez le coton, comprenant l'introduction du gène chimère selon l'une quelconque des revendications 3 à 6 ou du vecteur selon la revendication 7, dans le génome d'un plant de coton ; ou
la fourniture de la plante transgénique selon la revendication 9 ou 10.

14. Procédé selon la revendication 13, comprenant en outre la culture de ladite plante jusqu'à ce que des graines soient générées.

15. Utilisation du gène chimère selon l'une quelconque des revendications 3 à 6, du vecteur selon la revendication 7, ou de la plante transgénique selon la revendication 9 ou 10, pour une expression spécifique des graines d'un produit chez le coton ou pour altérer les propriétés des fibres chez le coton.
